(19) Europäisches Patentamt
European Patent Office
Office européen des brevets

(11) Veröffentlichungsnummer : **0 126 480 B1**

(12) # EUROPÄISCHE PATENTSCHRIFT

(45) Veröffentlichungstag der Patentschrift : **20.01.88**

(21) Anmeldenummer : **84105777.1**

(22) Anmeldetag : **21.05.84**

(51) Int. Cl.[4] : **C 07 D401/06**, C 07 D217/20, C 07 D217/26, A 61 K 31/47 // (C07D401/06, 217:20, 241:04),(C07D401/06, 217:20, 211:14)

(54) **Substituierte Isochinolinderivate, Verfahren zu deren Herstellung sowie Arzneimittel enthaltend diese Verbindungen.**

(30) Priorität : **19.05.83 US 496327**

(43) Veröffentlichungstag der Anmeldung : **28.11.84 Patentblatt 84/48**

(45) Bekanntmachung des Hinweises auf die Patenterteilung : **20.01.88 Patentblatt 88/03**

(84) Benannte Vertragsstaaten : **AT BE CH DE FR GB IT LI LU NL SE**

(56) Entgegenhaltungen :
**EP-A- 0 047 923**
**CHEMICAL ABSTRACTS, Vol. 91, No. 9, 27. August 1979, Columbus, Ohio, USA D. WALTEROVA et al. "Biotransformation of some 2-methylpapaverinium derivatives with rat liver enzymes in vitro" Seite 13, Spalte 2, Abstract-No. 68226n**
**CHEMICAL ABSTRACTS, Vol. 93, No. 3, 21. Juli 1980, Columbus, Ohio, USA Y. ICHINOHE et al. "A convenient synthesis of isoquinoline alkaloids using catalytic methods" Seite 722, Spalte 2, Abstract-No. 26598w**

(73) Patentinhaber : **F. HOFFMANN-LA ROCHE & CO. Aktiengesellschaft**
**CH-4002 Basel (CH)**

(72) Erfinder : **Bruderer, Hans, Dr.**
**Schulgasse 4**
**Biel-Benken (CH)**
Erfinder : **Kierstead, Richard Wightman**
**30 Willowbrook Drive**
**North Caldwell, NJ (US)**
Erfinder : **Mullin, John G., Jr.**
**519 Goffle Hill Road**
**Hawthorne, NJ (US)**
Erfinder : **Nakamura, Keiji**
**Shichirighama, 2-chome**
**1-35, Kamakura-shi Kanagawa-ken (JP)**
Erfinder : **O'Brien, Jay Philip**
**71 Ridge Road**
**Cedar Grove, NJ (US)**
Erfinder : **Tateishi, Mitsuru**
**Tobio-cho 1-chome**
**7-4, Atsugi-shi Kanagawa-ken (JP)**
Erfinder : **Teitel, Sidney**
**35 Allwood Place**
**Clifton, NJ (US)**

(74) Vertreter : **Lederer, Franz, Dr. et al**
**Van der Werth, Lederer & Riederer Patentanwälte**
**Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 126 480 B1**

Jouve, 18, rue St-Denis, 75001 Paris, France

**Beschreibung**

Die vorliegende Erfindung betrifft substituierte Isochinolinderivate der allgemeinen Formel

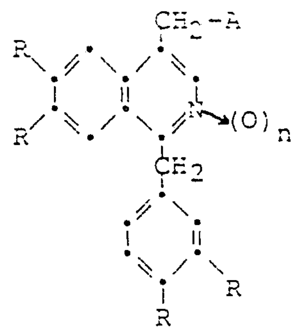

(I)

worin R nieder Alkoxy, n die Zahl 0 oder 1 und A eine der Gruppen

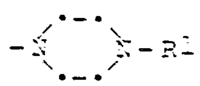

(a),

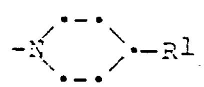

(b),

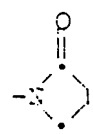

(c),

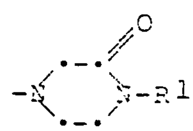

(d),

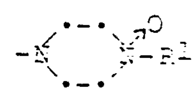

(e),

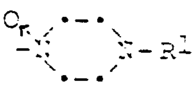

(f) und

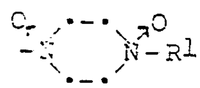

(g)

bedeuten, worin $R^1$ Phenyl, Halophenyl, nieder Alkylphenyl oder nieder Alkoxyphenyl bedeutet, und pharmazeutisch verwendbare Säureadditionssalze davon.

Der in dieser Beschreibung verwendete Ausdruck « nieder Alkyl » bezieht sich auf geradkettige oder verzweigte, gesättigte Kohlenwasserstoffreste mit 1-7 Kohlenstoffatomen, z. B. Methyl, Aethyl, Isopropyl, Butyl, Pentyl, Heptyl und dergleichen. Der Ausdruck « nieder Alkoxy » bezieht sich auf Alkyläthergruppen, in denen die nieder Alkylgruppe die obige Bedeutung besitzt, z. B. Methoxy, Aethoxy, Propoxy, Pentoxy und dergleichen. Die Ausdrücke « Halogen » und « HAL » beziehen sich auf die Halogene Brom, Chlor, Fluor und Jod. Beispiele von Halophenyl, nieder Alkylphenyl und nieder Alkoxyphenyl sind 2-Bromphenyl, 4-Fluorphenyl, 3-Methylphenyl, 2-Aethylphenyl, 4-Propylphenyl, 2-Aethoxyphenyl, 4-Butoxyphenyl, 3-Methoxyphenyl und dergleichen.

Eine bevorzugte Gruppe von Verbindungen der Formel I sind solche, worin n die Zahl 0 und A die obige Gruppe (a) bedeuten.

Eine bevorzugtere Gruppe von Verbindungen der Formel I sind solche, worin n die Zahl 0 und A die obige Gruppe (a) bedeuten, worin $R^1$ nieder Alkoxyphenyl bedeutet.

Eine speziell bevorzugte Verbindung der Formel I ist das 6,7-Dimethoxy-1-[(3,4-dimethoxyphenyl)methyl]-4-[[4-(2-methoxyphenyl)-1-piperazinyl]methyl]isochinolin.

Beispiele von Verbindungen der Formel I sind :

6,7-Diäthoxy-1-[(3,4-diäthoxyphenyl)methyl]-4-[[4-(2-methoxyphenyl)-1-piperazinyl]methyl]isochinolin ;

6,7-Dimethoxy-1-[(3,4-dimethoxyphenyl)methyl]-4-[[4-(3-chlorphenyl)-1-piperazinyl]methyl]isochinolin ;

6,7-Dipropoxy-1-[(3,4-dipropoxyphenyl)methyl]-4-[[4-(4-äthylphenyl)-1-piperazinyl]methyl]isochinolin ;

4-[(1-(3,4-Diäthoxybenzyl)-6,7-diäthoxy-4-isochinolinyl)methyl]-1-(2-methoxyphenyl)-2-piperazinon ;

1-[[6,7-Diäthoxy-1-[(3,4-diäthoxyphenyl)methyl]-4-isochinolinyl]methyl]-2-pyrrolidinon ;

1-(3,4-Diäthoxybenzyl)-6,7-diäthoxy-4-[[4′-(2-methoxyphenyl)-1′-piperazinyl]methyl]isochinolin-4-oxid ;

1-(3,4-Dimethoxybenzyl)-6,7-dimethoxy-4-[[4′-(2-chlorphenyl)-1′-piperazinyl]methyl]isochinolin-4′-oxid ;

1-(3,4-Diäthoxybenzyl)-6,7-diäthoxy-4-[[4′-(2-methoxyphenyl)-1′-piperazinyl]methyl]isochinolin-1′-oxid ;

1-(3,4-Dimethoxybenzyl)-6,7-dimethoxy-4-[[4'-(2-chlorphenyl)-1'-pipeprazinyl]methyl]isochinolin-1'-oxid ;

1-(3,4-Diäthoxybenzyl)-6,7-diäthoxy-4-[[4'-(2-methoxyphenyl)-1'-piperazinyl]methyl]isochinolin-1',4'-dioxid ;

1-(3,4-Dimethoxybenzyl)-6,7-dimethoxy-4-[[4'-(2-chlorphenyl)-1'-piperazinyl]methyl]isochinolin-1',4'-dioxid ;

1-(3,4-Diäthoxybenzyl)-6,7-diäthoxy-4-[[4'-(2-methoxyphenyl)-1'-piperazinyl]methyl]isochinolin-2,4'-dioxid ;

1-(3,4-Dimethoxybenzyl)-6,7-dimethoxy-4-[[4'-(2-chlorphenyl)-1'-piperazinyl]methyl]isochinolin-2,4'-dioxid und dergleichen.

Die Verbindungen der Formel I und deren pharmazeutisch verwendbare Säureadditionssalze können erfindungsgemäss hergestellt werden, indem man

a) zur Herstellung von Verbindungen der Formel I, worin A eine der obigen Gruppen (a), (b), (c), (d) und (e) bedeutet, eine Verbindung der allgemeinen Formel

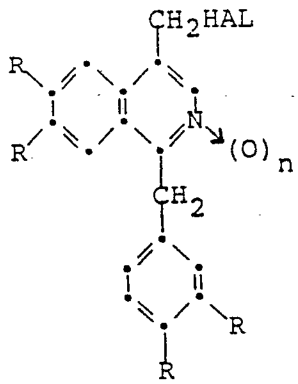

(II)

worin R und n die obige Bedeutung besitzen, mit einem Amin der allgemeinen Formel

HA' (III)

worin A' eine der obigen Gruppen (a), (b), (c), (d) und (e) bedeutet, umsetzt, oder

b) zur Herstellung von Verbindungen der Formel I, worin A eine der obigen Gruppen (e), (f) und (g) bedeutet, eine erhaltene Verbindung der Formel I, worin A die Gruppe (a) bedeutet, oxidiert und die erhaltenen N-Oxide aus dem Gemisch auftrennt, und

c) erwünschtenfalls, eine erhaltene Verbindung der Formel I in ein pharmazeutisch verwendbares Säureadditionssalz überführt.

Gemäss Verfahrensvariante a) wird ein 4-Halomethyl-6,7-dialkoxy-1-[(3,4-dialkoxyphenyl)methyl]isochinolin der Formel II durch Umsetzen mit einem Piperazin der allgemeinen Formel

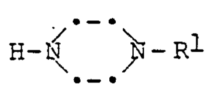

(IIIa)

worin $R^1$ die obige Bedeutung besitzt, oder einem Salz davon in ein 6,7-Dialkoxy-1-[(3,4-dialkoxyphenyl)methyl]-4-[(1-piperazinyl)methyl]isochinolin der Formel Ia übergeführt, d. h. in eine Verbindung der Formel I, worin A die obige Gruppe (a) bedeutet.

Die Piperazine der Formel IIIa sind bekannte Verbindungen oder können nach bekannten Methoden hergestellt werden. Beispiele solcher Verbindungen sind Phenylpiperazin, 1-(4-Chlorphenyl)piperazin, 1-(4-Tolyl)piperazin, 1-(4-Aethoxyphenyl)piperazin und dergleichen. Die Kondensation wird nach an sich bekannten Methoden durchgeführt, beispielsweise durch Erwärmen auf eine Temperatur zwischen etwa Raumtemperatur bis etwa 100° in Gegenwart eines inerten Lösungsmittels, wie Dimethylsulfoxid, Dimethylformamid und dergleichen, und in Gegenwart einer geeigneten Base, beispielsweise eines Alkalimetallhydroxids, wie Natriumhydroxid, Kaliumhydroxid und dergleichen, oder eines organischen Amins, beispielsweise eines Trialkylamins, wie Triäthylamin und dergleichen. Die erwünschte Verbindung der Formel Ia kann nach bekannten Methoden isoliert werden, beispielsweise durch Verdünnen mit Wasser und Filtrieren oder Extrahieren mit einem organischen Lösungsmittel, wie Methylenchlorid oder Aethylacetat, und anschliessendem Eindampfen.

Ein 4-Halomethyl-6,7-dialkoxy-1-[(3,4-dialkoxyphenyl)-methyl]isochinolin der Formel II wird durch Kondensation mit einem Piperidin der allgemeinen Formel

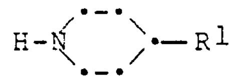

(IIIb)

worin $R^1$ die obige Bedeutung besitzt, oder einem Salz davon in ein 6,7-Dialkoxy-1-[(3,4-dialkoxyphenyl)methyl]-4-[(1-piperidinyl)-methyl]isochinolin der Formel Ib übergeführt, d. h. in eine Verbindung der Formel I, worin A die obige Gruppe (b) bedeutet.

Die Piperidine der Formel IIIb sind bekannte Verbindungen oder können nach bekannten Methoden hergestellt werden. Beispiele solcher Verbindungen sind : 4-Phenylpiperidin, 4-(4-Methoxyphenyl)piperidin, 4-(3-Tolyl)piperidin, 4-(2-Chlorphenyl)piperidin und dergleichen. Die Kondensation kann in ähnlicher Weise durchgeführt werden wie sie oben für die Herstellung einer Verbindung der Formel Ia beschrieben ist.

Ein 4-Halomethyl-6,7-dialkoxy-1-[(3,4-dialkoxyphenyl)-methyl]isochinolin der Formel II wird durch Kondensation mit einem Alkalimetallsalz von 2-Pyrrolidinon, einer bekannten Verbindung, in ein 6,7-Dialkoxy-1-[(3,4-dialkoxyphenyl)methyl]-4-[(1-pyrrolidinyl)methyl]isochinolin der Formel Ic übergeführt, d. h. in eine Verbindung der Formel I, worin A die obige Gruppe (c) bedeutet. Die Kondensation wird nach an sich bekannten Methoden durchgeführt, beispielsweise durch Erhitzen zum Rückfluss in einem organischen Lösungsmittel, wie Tetrahydrofuran und dergleichen. Die erwünschte Verbindung der Formel Ic kann nach an sich bekannten Methoden isoliert werden, beispielsweise durch Verdünnen mit Wasser, Extraktion mit einem organischen Lösungsmittel, wie Methylenchlorid oder Aethylacetat, und anschliessendem Eindampfen.

Ein 4-Halomethyl-6,7-dialkoxy-1-[(3,4-dialkoxyphenyl)methyl]isochinolin der Formel II wird durch Kondensation mit einem Piperazinon der allgemeinen Formel

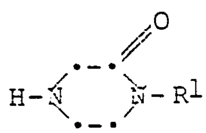

(IIId)

worin $R^1$ die obige Bedeutung besitzt, oder einem Salz davon in ein 6,7-Dialkoxy-1-[(3,4-dialkoxyphenyl)methyl]-4-[(1-piperazinyl-3-on)methyl]isochinolin der Formel Id übergeführt, d. h. in eine Verbindung der Formel I, worin A die obige Gruppe (d) bedeutet.

Die Piperazinone der Formel IIId sind bekannte Verbindungen oder können nach bekannten Methoden hergestellt werden. Beispiele solcher Verbindungen sind : 4-(4-Methoxyphenyl)-3-piperazinon, 4-(4-Tolyl)-3-piperazinon, 4-(3-Chlorphenyl)-3-piperazinon und dergleichen. Die Kondensation kann in ähnlicher Weise durchgeführt werden wie sie oben für die Herstellung von Verbindungen der Formel Ia beschrieben ist.

Schliesslich kann eine Verbindung der Formel Ie, d. h. eine Verbindung der Formel I, worin A die obige Gruppe (e) bedeutet, durch Umsetzen einer Verbindung der Formel II mit einem Piperazin-N-Oxid der allgemeinen Formel

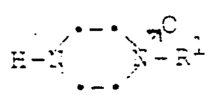

(IIIe)

worin $R^1$ die obige Bedeutung besitzt, oder einem Salz davon hergestellt werden.

Die Reaktionsbedingungen für diese Umsetzung sind die gleichen wie vorher für die Herstellung einer Verbindung der Formel Ia aus einer Verbindung der Formel II beschrieben. Die Piperazin-N-Oxide der Formel IIIe sind bekannte Verbindungen oder können nach bekannten Methoden hergestellt werden. Beispiele solcher Verbindungen sind : 1-(2-Methoxyphenyl)piperazin-1-oxid ; 1-(3-Chlorphenyl)piperazin-1-oxid ; 1-(4-Aethylphenyl)piperazin-1-oxid ; 1-(2-Aethoxyphenyl)piperazin-1-oxid ; 1-(3-Butylphenyl)piperazin-1-oxid ; 1-(2-Propoxyphenyl)piperazin-1-oxid und dergleichen.

Gemäss Verfahrensvariante B) können die Piperazin-N-Oxide der Formel Ie, If und Ig, d. h. die Verbindungen der Formel I, worin A eine der obigen Gruppen (e), (f) und (g) bedeutet, wie nachstehend beschrieben hergestellt werden. Demnach wird eine Verbindung der Formel Ia mit einem geeigneten Oxidationsmittel behandelt, z. B. Wasserstoffperoxid, Alkylhydroperoxide, Persäuren, wie m-Chlorperbenzoesäure und dergleichen. Die Oxidation kann unter bekannten Reaktionsbedingungen durchgeführt werden. Demnach kann die Oxidation bei einer Temperatur von etwa 0° bis etwa 5 °C in Gegenwart eines inerten organischen Lösungsmittels, wie Methylenchlorid und dergleichen, durchgeführt werden. Die erhaltene Reaktionsmischung besteht aus einem Gemisch der entsprechenden möglichen N-Oxide der Formeln

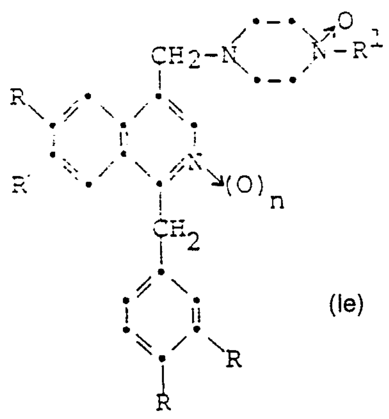

(Ie)

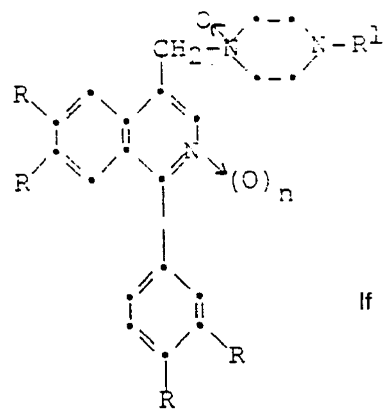

If und

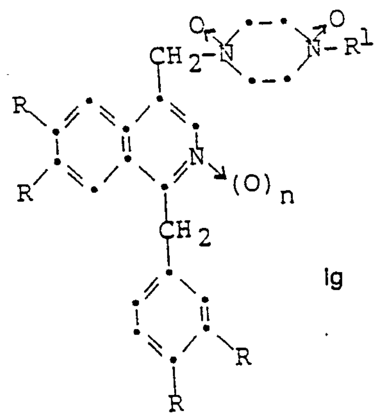

Ig

worin R, $R^1$ und n die obige Bedeutung besitzen. Jede der Verbindungen Ie, If und Ig kann nach bekannten Methoden, beispielsweise Chromatographie und dergleichen, aus dem Gemisch abgetrennt werden.

Die erfindungsgemässen Verbindungen der Formel I bilden Säureadditionssalze, und solche Salze sind ebenfalls Gegenstand der vorliegenden Erfindung. So bilden die Verbindungen der Formel I pharmazeutisch verwendbare Säureadditionssalze mit beispielsweise pharmazeutisch verwendbaren organischen und anorganischen Säuren, wie Essigsäure, Bernsteinsäure, Maleinsäure, Ameisensäure, Methansulfonsäure, p-Toluolsulfonsäure, Chlorwasserstoffsäure, Bromwasserstoffsäure, Salpetersäure, Phosphorsäure, Schwefelsäure und dergleichen. Die basischen Verbindungen der Formel I bilden Salze mit bis zu 3 Moläquivalenten Säure ; demnach bilden die basischen Verbindungen der Formel I Hemi-, Mono-, Di- oder Trisäuresalze.

Die Ausgangsmaterialien der Formel II sind neu und ebenfalls Gegenstand vorliegender Erfindung. Sie können wie im folgenden Schema und danach beschrieben hergestellt werden.

(Siehe Schema Seite 5 f.)

Schema

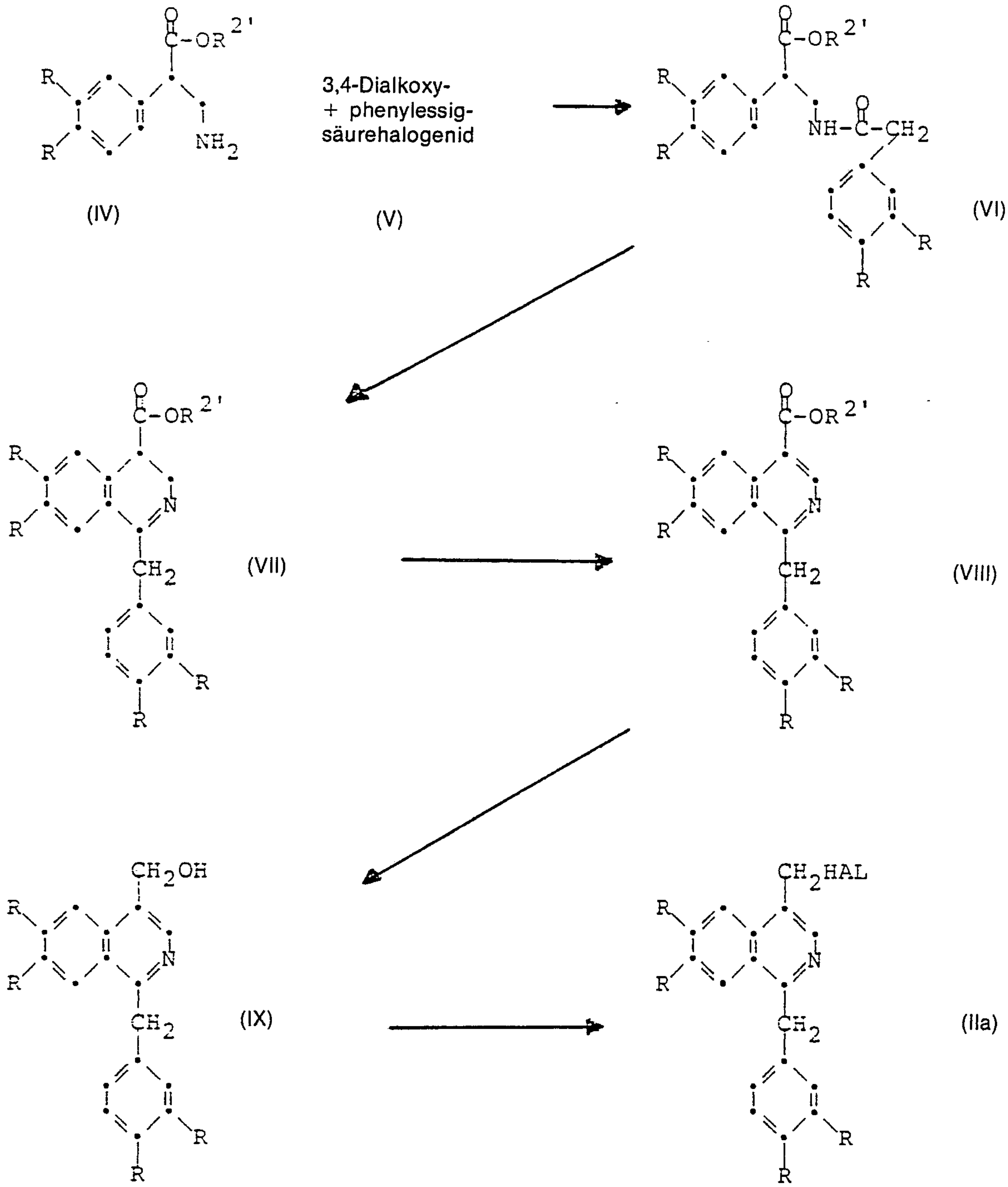


worin R und n die obige Bedeutung besitzen und HAL Halogen und $R^{2'}$ nieder Alkyl bedeuten.

Im Reaktionsschema wird eine Verbindung der Formel IV mit einer Verbindung der Formel V zu einer Verbindung der Formel VI kondensiert. Die Kondensation kann nach bekannten Methoden durchgeführt werden. Beispielsweise kann die Kondensation bei einer Temperatur im Bereich von etwa 0° bis etwa 40 °C, vorzugsweise bei 0 °C, in einem inerten organischen Lösungsmittel, wie Methylenchlorid und dergleichen, und in Gegenwart einer geeigneten Base, beispielsweise eines Alkalimetallhydroxids, wie Natriumhydroxid und dergleichen, durchgeführt werden. Die erhaltene Verbindung der Formel VI kann nach bekannten Methoden aus der organischen Phase, beispielsweise durch Eindampfen und dergleichen, erhalten werden.

Eine Verbindung der Formel VI kann nach bekannten Methoden zu einer Verbindung der Formel VII cyclisiert werden. So kann beispielsweise eine Verbindung der Formel VI mit einem Cyclisierungsmittel, wie Phosphoroxychlorid, in einem inerten organischen Lösungsmittel, wie Acetonitril und dergleichen, umgesetzt werden. Die erhaltene Verbindung der Formel VII kann nach bekannten Methoden, z. B. durch Eindampfen und dergleichen, isoliert werden.

Eine Verbindung der Formel VII kann zu einer Verbindung der Formel VIII dehydriert werden. Die Dehydrierung erfolgt nach bekannten Methoden, beispielsweise indem man ein gründlich durchgemischtes Gemisch einer Verbindung der Formel VII mit Schwefel erhitzt, wobei man die erwünschte Verbindung der Formel VIII erhält.

Eine Verbindung der Formel VIII kann zu einer Verbindung der Formel IX nach bekannten Methoden reduziert werden. Beispielsweise kann die Reduktion so ausgeführt werden, dass man eine Verbindung der Formel VIII in einem inerten Lösungsmittel mit einem geeigneten Reduktionsmittel, wie Natrium-bis-(2-methoxyäthoxy)aluminiumhydrid und dergleichen, behandelt. Das inerte Lösungsmittel kann beispielsweise Toluol, Tetrahydrofuran und dergleichen sein. Die erhaltene Verbindung der Formel IX kann durch Verdünnen des Reaktionsgemisches mit Wasser und Extraktion mit einem organischen Lösungsmittel, wie Methylenchlorid und dergleichen, erhalten werden. Danach wird das organische Lösungsmittel abgedampft, wobei man die erwünschte Verbindung der Formel IX erhält.

Gemäss einem Alternativverfahren kann der Ester der Formel VIII in die entsprechende Säure übergeführt werden, d. h. in eine Verbindung, die der Formel VIII entspricht, worin $R^{2'}$ jedoch Wasserstoff bedeutet, und zwar durch Hydrolyse unter Verwendung von beispielsweise Natriumhydroxid und dergleichen. Danach kann die erhaltene Säure unter Verwendung eines Reduktionsmittels, wie beispielsweise Diboran, in einem inerten organischen Lösungsmittel, beispielsweise Tetrahydrofuran und dergleichen, in den entsprechenden Alkohol der Formel IX übergeführt werden. Die gebildete Verbindung der Formel IX kann durch Verdünnen des Reaktionsgemisches mit Wasser und Extraktion mit einem organischen Lösungsmittel, wie Methylenchlorid und dergleichen, isoliert werden. Danach wird das organische Lösungsmittel eingedampft, wobei man die Verbindung der Formel IX erhält.

Eine Verbindung der Formel IX kann nach bekannten Methoden in das entsprechende Alkylhalogenid der Formel IIa übergeführt werden. Beispielsweise kann die Ueberführung durch Behandlung mit einem Thionylhalogenid in Gegenwart eines organischen Lösungsmittels, wie Methylenchlorid und dergleichen, bewerkstelligt werden. Die gebildete Verbindung der Formel IIa kann durch Verdünnen des erhaltenen Reaktionsgemisches mit Wasser und Extraktion mit einem organischen Lösungsmittel, wie Methylenchlorid und dergleichen, isoliert werden. Danach wird das organische Lösungsmittel eingedampft, wobei man die erwünschte Verbindung der Formel IIa erhält.

Eine Verbindung der Formel IIa kann nach bekannten Methoden in das entsprechende N-Oxid der allgemeinen Formel

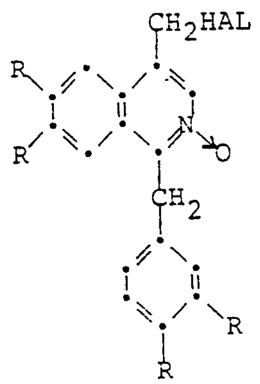

(IIb)

übergeführt werden. Insbesondere kann die N-Oxidation durch Behandlung einer Verbindung der Formel IIa mit einem geeigneten Oxidationsmittel, beispielsweise Wasserstoffperoxid, Alkylhydroperoxide, Persäuren, wie m-Chlorperbenzoesäure und dergleichen, bewerkstelligt werden. Die N-Oxidation kann nach bekannten Methoden durchgeführt werden. So kann die Oxidation bei einer Temperatur im Bereich von etwa 0° bis etwa 35 °C in Gegenwart eines inerten Lösungsmittels, beispielsweise Methylenchlorid und dergleichen, durchgeführt werden. Die erhaltene Verbindung der Formel IIb kann nach bekannten Methoden isoliert werden, beispielsweise durch Extraktion und Eindampfen und dergleichen.

Die α-(Aminomethyl)-3,4-di-nieder-alkoxybenzol-essigsäure-nieder-alkylester-Ausgangsmaterialien der Formel IV sind bekannte Verbindungen oder können nach bekannten Methoden hergestellt werden. Beispiele von Verbindungen der Formel IV sind :

α-(Aminomethyl)-3,4-dimethoxybenzol-essigsäure-äthyl-ester ;
α-(Aminomethyl)-3,4-diäthoxybenzol-essigsäure-äthyl-ester ;
α-(Aminomethyl)-3,4-dimethoxybenzol-essigsäure-methyl-ester ;
α-(Aminomethyl)-3,4-dibutoxybenzol-essigsäure-propyl-ester ;
α-(Aminomethyl)-3,4-dipropoxybenzol-essigsäure-methyl-ester und dergleichen.

Die 3,4-Dialkoxyphenyl-essigsäure-halogenid-Ausgangsmaterialien sind bekannte Verbindungen oder können nach bekannten Methoden hergestellt werden, die die Reaktion einer entsprechenden 3,4-Dialkoxyphenyl-essigsäure mit einem Thionylhalogenid, wie Thionylchlorid, in situ mitumfasst. Beispiele der 3,4-Dialkoxyphenyl-essigsäure-halogenid-Verbindungen sind :

3,4-Dimethoxyphenyl-essigsäurechlorid ;
3,4-Diäthoxyphenyl-essigsäurebromid ;
3,4-Dipropoxyphenyl-essigsäurechlorid ;
2,4-Dipentoxyphenyl-essigsäurechlorid und dergleichen.

Die Verbindungen der Formel I sowie deren pharmazeutisch verwendbare Säureadditionssalze sind wertvoll bei der Behandlung und Prophylaxe von cerebrovasculären Störungen, wie Hirnschlag, Cerebroarteriosklerose, vorübergehende ischämische Anfälle, Myocardischämie und Bluthochdruck.

So zeigt eine Verbindung der Formel I, nämlich das 6,7-Dimethoxy-1-[(3,4-dimethoxyphenyl)methyl]-4-[(4-(2-methoxyphenyl)-1-piperazinyl)methyl]isochinolin (nachstehend als Verbindung A bezeichnet), insbesondere eine verbesserte Erythrozytendeformabilität und selektive $\alpha_1$-Adrenoceptor-blockierende Wirkung. Im weiteren inhibiert die Verbindung A Erythrozytenmembranruptur, Lipidperoxidation und Blutplättchenaggregation.

Verbindung A übt ihre vaskuläre Wirkung über einen intrazellulären Hauptprozess auf die vaskuläre Kontraktion aus, indem sie die Bildung eines $Ca^{++}$-Calmodulin und Myosin light chain kinase-Komplexes hemmt. Demnach ist die Verbindung A von cerebralvasodilatierenden Calciumantagonisten, wie Nifedipin-Analogen, Nimodipin und Nicardipin, vollständig verschieden. Im Unterschied zu den meisten Vasodilatatoren ist die Verbindung A oral lang wirksam und verursacht keine Tachykardie und keinen orthostatischen Blutdruckabfall.

Der verminderte vertebrale und femorale vasculäre Widerstand, der durch die intravenöse Verabreichung einer Verbindung der Formel I hervorgerufen wird, kann wie nachstehend beschrieben gezeigt werden.

In anästhesierten Hunden bewirkt die Verbindung A nach Verabreichung in vier verschiedenen Dosen in die Femoralvene eine dosisabhängige (0,1-3 mg/kg) Verminderung des vasculären Widerstandes der Vertebral- und Femoralarterien und verursacht im Vergleich zu Papaverin eine 1,2 bzw. 1,9 mal höhere vertebrale bzw. femorale Vasodilatation. Die Resultate sind in den Tabellen I und II zusammengestellt. Die Verbindung A erniedrigt leicht und dosisabhängig (0,1-3 mg/kg i. v.) den diastolischen und systolischen Blutdruck, bewirkt jedoch keine reflektorische Tachykardie. Die Resultate für Verbindung A sowie andere erfindungsgemässe Verbindungen sind in Tabelle I zusammengefasst.

Die Wirkung von Verbindung A auf verschiedene Blutgefässe werden verglichen. Verbindung A (0,1-1,0 mg/kg i. v.) erhöht den regionalen cerebralen Blutfluss (parietaler Cortex) und vertebralen Blutfluss stärker als den Blutfluss in Femoral-, Mesenterial- und Renalgefässen. In Uebereinstimmung mit der arteriellen Verabreichung ist die vertebrale vasodilatierende Wirkung von Verbindung A nach intravenöser Verabreichung grösser als die vasodilatierende Wirkung auf die Carotis interna. Die vertebralen Blutflusswerte sind bei Hunden äquivalent zu den Blutflusswerten in der Carotis interna (30 ml/Min.). Die coronare vasodilatierende Wirkung der Verbindung ist vergleichbar mit deren vertebraler vasodilatierender Wirkung.

Die Verminderung des vertebralen und femoralen vasculären Widerstandes, welcher durch die intraduodenale Verabreichung einer Verbindung der Formel I hervorgerufen wird, kann wie nachstehend beschrieben gezeigt werden.

Die Testverbindungen werden in 0,1 %iger Natriumcarboxymethylcellulose-Lösung mittels eines implantieren Katheters mit Pentobarbital anästhesierten Hunden intraduodenal appliziert. Verbindung A vermindert dosisabhängig (3, 10 und 30 mg/kg i. d.) den vertebralen vasculären Widerstand särker als den femoralen vasculären Widerstand. Parallel zur vertebralen Vasodilatation setzt Verbindung A den diastolischen/systolischen Blutdruck und die Herzfrequenz leicht herab. Die Resultate sind ebenfalls in Tabelle I zusammengefasst.

Die vertebrale vasodilatierende Wirkung von Verbindung A ist selektiver und länger wirksam als diejenige nach intravenöser und intraarterieller Verabreichung.

(Siehe Tabellen Seite 9 ff.)

Tabelle I

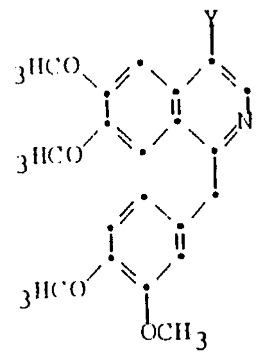

| Verbindungen Y | | Aenderung in % 1.0 mg/kg i.v. s-BP/d-BP (Δ mmHg) | HR (Δ Schläge/min) | 10 mg/kg i.d. VVR | FVR | s-BP/d-BP (Δ mmHg) | HR (Δ Schläge/min) |
|---|---|---|---|---|---|---|---|
| A | 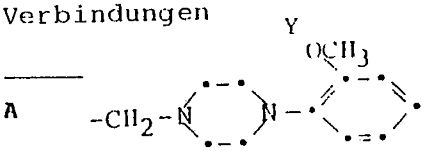 | -30/-41 | -2 | -17 | -9 | -17/-24 | -7 |
| B | 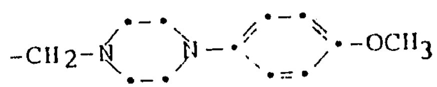 | -16/-23 | 8 | – | – | – | – |
| C | 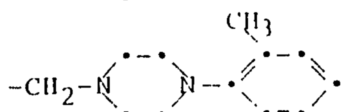 | -15/-16 | 6 | – | – | – | – |
| D | 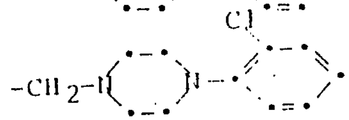 | -14/-12 | 14 | – | – | – | – |
| E | 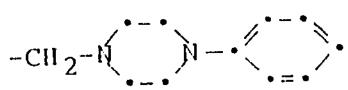 | -6/-4 | 3 | – | – | – | – |

VVR : Vertebraler Vasculärer Widerstand
FVR : Femoraler Vasculärer Widerstand

BP : Blutdruck
HR : Herzfrequenz

Tabelle I (Fortsetzung)

| Verbindungen | Y | Aenderung in % 1.0 mg/kg i.v. s-BP/d-BP (Δ mmHg) | HR (ΔSchläge/min) | 10 mg/kg i.d. VVR | FVR | s-BP/d-BP (Δ mmHg) | HR (ΔSchläge/min) |
|---|---|---|---|---|---|---|---|
| F | 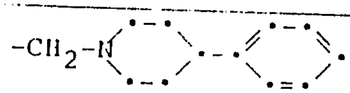 | -4/-4 | 6 | - | - | - | - |
| G | 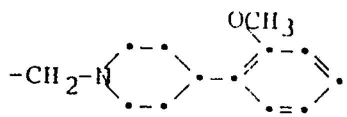 | -24/-32 | 6 | -10 | -8 | -12/-17 | 10 |
| H | 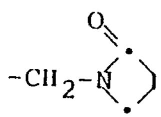 | -18/-23 | 7 | -3 | -5 | -1/-8 | 18 |
| Papaverin | | -18/-34 | 23 | -11 | -7 | -11/-14 | 15 |

VVR : Vertebraler Vasculärer Widerstand
FVR : Femolarer Vasculärer Widerstand

BP : Blutdruck
HR : Herzfrequenz

Tabelle II

Vasodilatierender Effekt einer erfindungsgemässen Verbindung und Papaverin

| Verbindung | Aenderung im VVR (10 mg/kg i.d.) | | VVR -Potenz gegenüber Papaverin (iv) | Aenderung in FVR (10 mg/kg i.d.) | | FVR-Potenz gegenüber Papaverin (iv) |
|---|---|---|---|---|---|---|
| | % Aenderung | Dauer (min) | | % Aenderung | Dauer (min) | |
| Papaverin | -11 | 60 | 1.0 | -7 | 40 | 1.0 |
| Verbindung A | -17 | >180 | 1.23 | -9 | >180 | 1.93 |

Eine erfindungsgemässe Verbindung der Formel I oder ein pharmazeutisch verwendbares Säureadditionssalz davon kann zusammen mit üblichen pharmazeutischen Hilfsstoffen, beispielsweise organischen oder anorganischen inerten Trägermaterialien wie Wasser, Gelatine, Lactose, Stärke, Magnesiumstearat, Talk, pflanzliche Oele, Gummi, Polyalkylenglykole und dergleichen in pharmazeutische Standarddosierungsformen eingearbeitet werden, welche für die orale oder parenterale Anwendung geeignet sind. Die pharmazeutischen Präparate können in fester Form, beispielsweise als Tabletten, Lutschtabletten, Suppositorien, Kapseln, oder in flüssiger Form, z. B. als Lösungen, Suspensionen oder Emulsionen, verwendet werden. Die pharmazeutischen Hilfsstoffe umfassen auch Konservierungsmittel, Stabilisierungsmittel, Netzmittel oder Emulgiermittel, Salze zum Variieren des osmotischen Druckes oder Puffer. Die pharmazeutischen Präparate können auch noch andere therapeutisch aktive Verbindungen enthalten.

Die Menge an aktivem Medikament, d. h. an einer Verbindung der Formel I oder einer äquivalenten Menge eines Salzes davon, welche in allen oben beschriebenen Dosierungsformen vorhanden sein kann, ist variabel. Es ist jedoch bevorzugt, Kapseln oder Tabletten zur Verfügung zu stellen, welche ungefähr 30 bis ungefähr 300 mg basische Verbindung der Formel I oder eine äquivalente Menge eines pharmazeutisch verwendbaren Salzes davon enthalten. Für die parenterale Verabreichung ist es bevorzugt, eine Lösung zur Verfügung zu stellen, welche ungefähr 10 mg/ml bis ungefähr 50 mg/ml der basischen Verbindung der Formel I oder der äquivalenten Menge eines Salzes davon, enthält. Die Häufigkeit, mit welcher diese Dosierungsformen Warmblütlern verabreicht werden, können variiert werden in Abhängigkeit von der in der Dosierungsform vorhandenen Menge an aktivem Medikament und den Bedürfnissen und Verlangen des Patienten. Gewöhnlich werden jedoch bis zu ungefähr 10 mg/kg einer Verbindung der Formel I oder einer äquivalenten Menge eines Salzes davon täglich in mehreren Teildosen verabreicht. Es sei jedoch an dieser Stelle betont, dass die oben genannten Dosierungen lediglich als Beispiele angegeben wurden und dass sie den Umfang der vorliegenden Erfindung in keiner Weise beschränken.

Die nachfolgenden Beispiele veranschaulichen die vorliegende Erfindung. Sofern nicht anders erwähnt, sind alle Temperaturen in Celsius Graden angegeben.

## Beispiel 1

Herstellung von 2-(3,4-Dimethoxyphenyl)-3-//[(3,4-dimethoxyphenyl) methyl] carbonyl/amino/propanonsäureäthylester

Ein Gemisch von 289 g α-(Aminomethyl)-3,4-dimethoxybenzolessigsäureäthylester-hydrochlorid, 1 000 ml Methylenchlorid und 1 000 ml Wasser werden auf 0° abgekühlt und mit 4N Natriumhydroxydlösung auf pH 9 eingestellt. Eine Lösung von 1 000 ml Methylenchlorid und 3,4-Dimethoxyphenylessigsäurechlorid (vorgängig durch Umsetzen von 400 ml Thionylchlorid und 215 g 3,4-Dimethoxyphenylessigsäure in 500 ml Toluol bei 50° während 1 Stunde und Eindampfen zur Trockene hergestellt) wird gleichzeitig mit einer 4N Natriumhydroxydlösung zugegeben, wobei auf diese Weise ein pH von 7,0-8,0 beibehalten wird. Nach beendeter Zugabe des Säurechlorids wird der pH während 1 Stunde bei 9,0 gehalten, und danach wird mit 6N Salzsäure auf pH 1,0 angesäuert. Die Schichten werden getrennt, und die wässrige Phase einmal mit Methylenchlorid extrahiert. Die vereinigten organischen Phasen werden einmal mit einem Ueberschuss an gesättigter Natriumbicarbonatlösung gewaschen, über Natriumsulfat getrocknet und unter vermindertem Druck eingedampft, wobei man 430 g 2-(3,4-Dimethoxyphenyl)-3-//[(3,4-dimethoxyphenyl methyl] carbonyl/amino/propanonsäureäthylester als Oel erhält.

## Beispiel 2

Herstellung von 2-(3,4-Dimethoxyphenyl)-3-//[(3,4-dimethoxyphenyl) methyl] carbonyl/amino/propanonsäure

Ein Gemisch von 18,4 g 2-(3,4-Dimethoxyphenyl)-3-//[(3,4-dimethoxyphenyl) methyl] carbonyl/amino/propanonsäureäthylester, 50 ml Aethanol und 65 ml 10 %iger Natriumhydroxydlösung wird während 2 Stunden zum Rückfluss erhitzt, danach zur Entfernung des Aethanols eingeengt und mit Wasser verdünnt. Die wässrige Phase wird mit Aethylacetat gewaschen, mit Ueberschuss an 6N Salzsäure angesäuert, und das Produkt mit Aethylacetat extrahiert, getrocknet und eingedampft. Anreiben des Rückstandes mit Aether liefert 10,9 g unlösliche 2-(3,4-Dimethoxyphenyl)-3-//[(3,4-dimethoxyphenyl) methyl] carbonyl/amino/propansäure, Schmelzpunkt 151-153°. Kristallisation aus Aethylacetat liefert eine Analysenprobe vom Schmelzpunkt 150-152°.

Analyse für $C_{21}H_{55}NO_7$

| | | | |
|---|---|---|---|
| Berechnet : | C 62,52 | H 6,25 | N 3.47. |
| Gefunden : | C 62,41 | H 6,26 | N 3.45. |

## Beispiel 3

Herstellung von 2-(3,4-Dimethoxyphenyl)-3-//[(3,4-dimethoxyphenyl) methyl] carbonyl/amino/propanonsäuremethylester

Eine Lösung von 23,3 g 2-(3,4-Dimethoxyphenyl)-3-//[(3,4-dimethoxyphenyl) methyl] carbonyl/amino/propansäure und 15,5 ml konzentrierter Schwefelsäure in 300 ml Methanol wird während 6 Stunden zum Rückfluss erhitzt und danach zur Trockene eingedampft. Der Rückstand wird zwischen Aethylacetat und verdünnter Natriumbicarbonatlösung verteilt, und die organische Phase wird getrocknet und eingedampft, wobei man 15,5 g rohen 2-(3,4-Dimethoxyphenyl)-3-//[(3,4-dimethoxyphenyl) methyl] carbonyl/amino/propanonsäuremethylester erhält. Kristallisation aus Aethylacetat liefert eine Analysenprobe vom Schmelzpunkt 92-93°.

Analyse für $C_{22}H_{27}NO_7$
Berechnet : C 63,30 H 6,52 N 3,36
Gefunden : C 63,35 H 6,52 N 3.31.

Beispiel 4

Herstellung von 3,4-Dihydro-6,7-dimethoxy-1-[(3,4-dimethoxyphenyl) methyl]-4-isochinolincarbonsäureäthylester-hydrochlorid

Ein Gemisch von 430 g (1,0 Mol) 2-(3,4-Dimethoxyphenyl)-3-//[(3,4-dimethoxyphenyl) methyl] carbonyl/amino/propansäureäthylester, 4 300 ml Acetonitril und 430 g Phosphoroxychlorid wird während 1 Stunde zum Rückfluss erhitzt und danach unter vermindertem Druck eingedampft. Kristallisation des Rückstandes aus Aethanol liefert 399 g des Hydrochlorids von 3,4-Dihydro-6,7-dimethoxy-1-[(3,4-dimethoxyphenyl) methyl]-4-isochinolincarbonsäureäthylester als gelben Feststoff, Schmelzpunkt 213-214° (Zers.).

Analyse für $C_{23}H_{27}NO_6 \cdot HCl$
Berechnet : C 61,40 H 6,27 N 3,11 Cl 7,88
Gefunden : C 61,11 H 6,48 N 3,06 Cl 7.89.

Beispiel 5

Herstellung von 3,4-Dihydro-6,7-dimethoxy-1-[(3,4-dimethoxyphenyl) methyl]-4-isochinolincarbonsäuremethylester-hydrochlorid

Das Hydrochlorid von 3,4-Dihydro-6,7-dimethoxy-1-[(3,4-dimethoxyphenyl) methyl]-4-isochinolincarbonsäuremethylester wird mit der in Beispiel 4 für die Herstellung von 3,4-Dihydro-6,7-dimethoxy-1-[(3,4-dimethoxyphenyl) methyl]-4-isochinolincarbonsäureäthylester-hydrochlorid beschriebenen Methode aus 2-(3,4-Dimethoxyphenyl)-3-//[(3,4-dimethoxyphenyl) methyl] carbonyl/amino/propansäuremethylester hergestellt. Kristallisation aus Methanol liefert analysenreines 3,4-Dihydro-6,7-dimethoxy-1-[(3,4-dimethoxyphenyl) methyl]-4-isochinolincarbonsäuremethylester-hydrochlorid, Schmelzpunkt 199-200°.

Analyse für $C_{22}H_{25}NO_6 \cdot HCl$
Berechnet : C 60,62 H 6,01 N 3,21
Gefunden : C 60,56 H 6,08 N 3,17.

Beispiel 6

Herstellung von 3,4-Dihydro-6,7-dimethoxy-1-[(3,4-dimethoxyphenyl) methyl]-4-isochinolincarbonsäure

Ein Gemisch von 3 g 3,4-Dihydro-6,7-dimethoxy-1-[(3,4-dimethoxyphenyl) methyl]-4-isochinolincarbonsäureäthylester-hydrochlorid in 15 ml Aethanol und 15 ml 10 %iger Natriumhydroxydlösung wird während 1 Stunde zum Rückfluss erhitzt und danach zur Entfernung des Aethanols eingeengt. Der wässrige Rückstand wird mit 6N Salzsäure auf pH 6,7 eingestellt, und das ausgefallene Produkt abfiltriert. Trocknen liefert 1,9 g 3,4-Dihydro-6,7-dimethoxy-1-[(3,4-dimethoxyphenyl) methyl]-4-isochinolincarbonsäure, Schmelzpunkt 154-155°.

Beispiel 7

Herstellung von 6,7-Dimethoxy-1-[(3,4-dimethoxyphenyl)-methyl]-4-isochinolincarbonsäureäthylester

Die freie Base von 3,4-Dihydro-6,7-dimethoxy-1-[(3,4-dimethoxyphenyl) methyl]-4-isochinolincarbonsäureäthylester hydrochlorid wird unmittelbar vor dem Gebrauch hergestellt, indem man 135 g 3,4-Dihydro-6,7-dimethoxy-1-[(3,4-dimethoxyphenyl) methyl]-4-isochinolincarbonsäureäthylester-hydrochlorid zwischen Methylenchlorid und gesättigter Natriumbicarbonatlösung verteilt, über Natriumsulfat trocknet und vom Aether abdampft, wobei man einen Aether-solvatisierten weissen Festkörper erhält. Sublimierter Schwefel (10,5 g) wird zugegeben, und das erhaltene Gemisch unter einem langsamen Strom von Argon bei 150° unter Bildung von Schwefelwasserstoff erhitzt, bis man eine klare geschmolzene Masse erhält. Das Gemisch wird dann auf etwa 80° abgekühlt, in heissem Aethanol gelöst und mit vier Teilen Aether verdünnt. Das gekühlte Gemisch wird filtriert, wobei man 98,6 g 6,7-Dimethoxy-1-[(3,4-dimethoxyphenyl) methyl]-4-isochinolincarbonsäureäthylester als weissen Festkörper erhält, Schmelzpunkt 117-119°. Umkristallisation aus Aether liefert eine Analysenprobe vom Schmelzpunkt 117-119°.

Analyse für $C_{23}H_{25}NO_6$

| | | | |
|---|---|---|---|
| Berechnet : | C 67,14 | H 6,12 | N 3,40 |
| Gefunden : | C 67,17 | H 6,19 | N 3,53. |

Beispiel 8

Herstellung von 6,7-Dimethoxy-1-[(3,4-dimethoxyphenyl)-methyl]-4-isochinolincarbonsäuremethylester und dessen Hydrobromidsalz

Ein Gemisch von 3,9 g 3,4-Dihydro-6,7-dimethoxy-1-[(3,4-dimethoxyphenyl) methyl]-4-isochinolincarbonsäuremethylester-hydrochlorid, 100 ml Decalin und 2 g 10 %igem Palladium auf Kohle wird unter Rühren während 2 Stunden zum Rückfluss erhitzt. Man verdünnt das abgekühlte Gemisch mit Wasser, säuert es mit 6N Salzsäure an und filtriert den Katalysator ab. Die wässrige Lösung wird mit Aether gewaschen, mit überschüssigem Ammoniumhydroxyd basisch gestellt, und das Produkt mit Aethylacetat extrahiert, getrocknet und eingedampft, wobei man 4,1 g rohen 6,7-Dimethoxy-1-[(3,4-dimethoxyphenyl) methyl]-4-isochinolincarbonsäuremethylester erhält. Kristallisation aus Aether liefert analysenreines Material vom Schmelzpunkt 127-128°.

Analyse für $C_{22}H_{23}NO_6$

| | | | |
|---|---|---|---|
| Berechnet : | C 66,49 | H 5,83 | N 3,52 |
| Gefunden : | C 66,79 | H 6,00 | N 3,51. |

1,5 g der freien Base von 6,7-Dimethoxy-1-[(3,4-dimethoxyphenyl) methyl]-4-isochinolincarbonsäuremethylester in Aethanol wird mit einer Lösung äthanolischer Bromwasserstoffsäure umgesetzt, und der ausgefallene Festkörper abfiltriert. Umkristallisation aus Aethanol liefert 1,8 g analysenreines Hydrobromidsalz vom Schmelzpunkt 188-189°.

Analyse für $C_{22}H_{23}NO_6 \cdot HBr$

| | | | |
|---|---|---|---|
| Berechnet : | C 55,24 | H 5,05 | N 2.93 |
| Gefunden : | C 55,19 | H 5,08 | N 2.90. |

Beispiel 9

Herstellung von 6,7-Dimethoxy-1-[(3,4-dimethoxyphenyl)-methyl]-4-isochinolincarbonsäure und dessen Hydrobromidsalz

2,0 g 6,7-Dimethoxy-1-[3,4-dimethoxyphenyl) methyl]-4-isochinolincarbonsäuremethylester, 20 ml Aethanol und 20 ml 10 %ige Natriumhydroxydlösung werden unter Rühren während 2 Stunden zum Rückfluss erhitzt und zur Entfernung des Aethanols eingedampft. Der Rückstand in Wasser wird mit Bromwasserstoffsäure angesäuert und dreimal mit Chloroform extrahiert, getrocknet und eingedampft. Kristallisation des Rückstandes aus Acetonitril liefert 1,8 g rohes Hydrobromidsalz, Schmelzpunkt 210-211°.

Ein Teil des rohen Hydrobromidsalzes in wässriger Ammoniumhydroxydlösung wird mit 6N Salzsäure auf pH 6,7 eingestellt, und der Festkörper abfiltriert, wobei man analysenreine freie Base von 6,7-Dimethoxy-1-[(3,4-dimethoxyphenyl) methyl]-4-isochinolincarbonsäure vom Schmelzpunkt 235-236° erhält.

Analyse für $C_{21}H_{21}NO_6$

| | | | |
|---|---|---|---|
| Berechnet : | C 65,78 | H 5,52 | N 3.65 |
| Gefunden : | C 65,72 | H 5,35 | N 3,47. |

Beispiel 10

Herstellung von 6,7-Dimethoxy-1-[(3,4-dimethoxyphenyl)-methyl]-4-isochinolinmethanol und dessen Hydrobromidsalz

Eine Aufschlämmung von 41,1 g 6,7-Dimethoxy-1-[(3,4-dimethoxyphenyl) methyl]-4-isochinolincarbonsäureäthylester in 250 ml trockenem Tetrahydrofuran wird auf 0° abgekühlt und tropfenweise während 30 Minuten mit 62,8 ml einer 3,5 M Lösung von Natrium-bis-(2-methoxyäthoxy) aluminiumhydrid in Toluol versetzt. Nach 3 Stunden Rühren bei 0° werden 60 ml einer gesättigten wässrigen Natriumsulfatlösung vorsichtig zugegeben, und das Gemisch bis zur Granulierung gerührt. Die überstehende Lösung wird abdekantiert, der Rückstand mit warmem Methylenchlorid extrahiert, und die vereinigten organischen Lösungen unter vermindertem Druck eingedampft. Der Rückstand wird in Methylenchlorid gelöst, mit Wasser gewaschen, über Natriumsulfat getrocknet und eingedampft. Kristallisation aus Methylenchlorid/Aethylacetat liefert 26,7 g 6,7-Dimethoxy-1-[(3,4-dimethoxyphenyl) methyl]-4-isochinolinmethanol als weissen Festkörper, Schmelzpunkt 140-146°.

Ein Teil der Base von 6,7-Dimethoxy-1-[(3,4-dimethoxyphenyl) methyl]-4-isochinolinmethanol in Aethanol wird mit 1,1 Moläquivalenten äthanolischer Bromwasserstoffsäurelösung behandelt und filtriert, wobei man analysenreines Hydrobromidsalz vom Schmelzpunkt 210-212° erhält.

Analyse für $C_{21}H_{23}NO_5 \cdot HBr$
Berechnet : C 56,01 H 5,37 N 3,11
Gefunden : C 56,10 H 5,26 N 3.06.

Das 6,7-Dimethoxy-1-[(3,4-dimethoxyphenyl) methyl]-4-isochinolinmethanol kann auch wie folgt hergestellt werden :

Eine Aufschlämmung von 25,2 g 6,7-Dimethoxy-1-[(3,4-dimethoxyphenyl) methyl]-4-isochinolincarbonsäure in 100 ml trockenem Tetrahydrofuran wird bei 15° in einer Inertgasatmosphäre gerührt, während vorsichtig 200 ml 1 molarer Boran/Tetrahydrofurankomplex zugegeben werden. Nach 2-stündigem Rühren bei Raumtemperatur hat sich der ursprüngliche Festkörper gelöst und ist durch einen zweiten Niederschlag ersetzt. Das Gemisch wird vorsichtig mit 200 ml Wasser versetzt, und das Tetrahydrofuran unter vermindertem Druck bei 40° entfernt. Die abgekühlte Reaktionsmischung wird dann filtriert, mit Wasser gewaschen und der feuchtigkeitsstabile Borankomplex unter Rühren portionenweise zu 200 ml 2N Salzsäure zugegeben, die vorgängig auf 70-80° erwärmt wurde. Die erhaltene Lösung wird während weiteren 15 Minuten auf einem Dampfbad erwärmt und danach abgekühlt und mit 100 ml Diäthyläther extrahiert. Die wässrige Lösung wird tropfenweise zu 250 ml einer kalten 2N Natriumhydroxydlösung unter Rühren zugegeben. Der erhaltene Festkörper wird abfiltriert, mit Wasser gut gewaschen und getrocknet, wobei man 20,6 g rohes 6,7-Dimethoxy-1-[(3,4-dimethoxyphenyl)-methyl]-4-isochinolinmethanol erhält, Schmelzpunkt 146-148°. Umkristallisation aus Methylenchlorid/Aethylacetat liefert 19,2 g gereinigtes 6,7-Dimethoxy-1-[(3,4-dimethoxyphenyl) methyl]-4-isochinolinmethanol vom Schmelzpunkt 141-143°.

## Beispiel 11

Herstellung von 4-Chlormethyl-6,7-dimethoxy-1-[(3,4-dimethoxyphenyl) methyl] isochinolin

Ein gekühltes Gemisch von 26,8 g 6,7-Dimethoxy-1-[(3,4-dimethoxyphenyl) methyl]-4-isochinolinmethanol in 600 ml Methylenchlorid wird innert 15 Minuten tropfenweise mit 27 ml Thionylchlorid versetzt. Das Kühlbad wird entfernt, und die Lösung während 3 Stunden auf Raumtemperatur aufgewärmt, und danach werden die flüchtigen Anteile unter vermindertem Druck entfernt. Der Rückstand wird mit Methylenchlorid und Wasser gemischt, wonach vorsichtig überschüssiges Natriumbicarbonat zugegeben wird. Die organische Phase wird abgetrennt, über Natriumsulfat getrocknet, eingedampft und aus Aethylacetat kristallisiert, wobei man 23,1 g 4-Chlormethyl-6,7-dimethoxy-1-[(3,4-dimethoxyphenyl) methyl] isochinolin als gelben Festkörper erhält, Schmelzpunkt 153° (Zers.). Umkristallisation aus Aethylacetat liefert analysenreines Material vom Schmelzpuntk 153° (Zers.).

Analyse für $C_{21}H_{22}ClNO_4$
Berechnet : C 65,03 H 5,72 N 3,61 Cl 9,14
Gefunden : C 64,74 H 5,82 N 3,91 Cl 9,20.

## Beispiel 12

Herstellung von 6,7-Dimethoxy-1-[(3,4-dimethoxyphenyl)-methyl]-4-[(4-phenyl-1-piperazinyl) methyl] isochinolin und dessen Dihydrochlorid-hemihydratsalz

Ein Gemisch von 5,0 g 4-Chlormethyl-6,7-dimethoxy-1-[(3,4-dimethoxyphenyl) methyl] isochinolin, 2,1 g Phenylpiperazin und 2,63 g Triäthylamin in 50 ml Dimethylsulfoxyd wird während 1 Stunde unter Argon auf 80° erhitzt, und danach wird das abgekühlte Gemisch mit Wasser verdünnt. Der Festkörper wird abfiltriert, in Methylenchlorid gelöst und mit gesättigter Natriumbicarbonatlösung gewaschen, über Natriumsulfat getrocknet, mit Kohle behandelt und eingedampft. Der Rückstand wird aus Aethanol kristallisiert, wobei man 5,3 g 6,7-Dimethoxy-1-[(3,4-dimethoxyphenyl) methyl]-4-[(4-phenyl-1-piperazinyl) methylisochinolin erhält, Schmelzpunkt 183-184°. Umkristallisation aus Aethanol liefert eine Analysenprobe vom Schmelzpunkt 183-184°.

Analyse für $C_{31}H_{35}N_3O_4$
Berechnet : C 72,49 H 6,87 N 8,18
Gefunden : C 72,30 H 6,85 N 8,21.

5,0 g der freien Base von 6,7-Dimethoxy-1-[(3,4-dimethoxyphenyl) methyl]-4-[(4-phenyl-1-piperazinyl) methyl] isochinolin in 50 ml warmem Aethanol werden mit 2 Moläquivalenten äthanolischer Salzsäurelösung versetzt, und der erhaltene Festkörper abfiltriert. Umkristallisation aus 95 %igem Aethanol und

Trocknen des Produktes bei 80°/13 Pa über Phosphorpentoxyd liefert 5,3 g analysenreines hydratisiertes Dihydrochloridsalz vom Schmelzpunkt 217-219° (Zers.).

Analyse für $C_{21}H_{35}N_3O_4 \cdot 2HCl \cdot 0,5\ H_2O$
Berechnet: C 62,52 H 6,43 N 7,06 Cl 11,91 $H_2O$ 1.51
Gefunden: C 62,59 H 6,33 N 7,05 Cl 12,33 $H_2O$ 1,49.

Beispiel 13

Herstellung von 6,7-Dimethoxy-1-/(3,4-dimethoxyphenyl)-methyl/4-/[4-(2-chlorphenyl)-1-piperazinyl] methyl/isochinolin und dessen Dihydrochlorid-1,5-molares-hydratsalz

Ein Gemisch von 5,0 g 4-Chlormethyl-6,7-dimethoxy-1-[(3,4-dimethoxyphenyl) methyl] isochinolin, 3,5 g 1-(2-Chlorphenyl) piperazin-dihydrochlorid und 5,26 g Triäthylamin in 50 ml Dimethylsulfoxyd wird während 1 Stunde unter Argon auf 80° erhitzt, und dann wird das abgekühlte Gemisch mit Wasser verdünnt. Der Festkörper wird abfiltriert, in Methylenchlorid gelöst und mit gesättigter Natriumbicarbonatlösung gewaschen, über Natriumsulfat getrocknet, mit Kohle behandelt und eingedampft. Der Rückstand wird aus Aethanol kristallisiert, wobei man 5,9 g 6,7-Dimethoxy-1-/(3,4-dimethoxyphenyl) methyl/-4-/[4-(2-chlorphenyl)-1-piperazinyl] methyl/isochinolin als braunen Festkörper erhält, Schmelzpunkt 141-142°. Umkristallisation aus Aethanol liefert eine Analysenprobe vom Schnelzpunkt 141-142°.

Analyse für $C_{31}H_{34}ClN_3O_4$
Berechnet: C 67,94 H 6,25 N 7,67 Cl 6,47
Gefunden: C 67,95 H 6,34 N 7,77 Cl 6,69.

5,2 g der freien Base von 6,7-Dimethoxy-1-/(3,4-dimethoxyphenyl) methyl/-4-/[4-(2-chlorphenyl)-1-piperazinyl] methyl/isochinolin in 50 ml warmem Aethanol werden mit 2,2 Moläquivalenten äthanolischer Salzsäurelösung versetzt, und der erhaltene Festkörper abfiltriert. Umkristallisation aus 95 %igem Aethanol liefert 6,0 g analysenreines hydratisiertes Dihydrochloridsalz vom Schmelzpunkt 227-228°.

Analyse für $C_{31}H_{34}ClN_3O_4 \cdot 2HCl \cdot 1,5\ H_2O$
Berechnet: C 57,46 H 6,07 N 6,48 Cl 16,41 $H_2O$ 4,17
Gefunden: C 57,22 H 5,83 N 6,43 Cl 16,02 $H_2O$ 4,21.

Beispiel 14

Herstellung von 6,7-Dimethoxy-1-/(3,4-dimethoxyphenyl)-methyl/-4-/[4-(2-methylphenyl)-1-piperazinyl] methyl/isochinolin und dessen Dihydrochlorid-1,25-molares-hydratsalz

Ein Gemisch von 5,0 g 4-Chlormethyl-6,7-dimethoxy-1-[(3,4-dimethoxyphenyl) methyl] isochinolin, 3,24 g 1-(2-Methylphenyl) piperazin-dihydrochlorid und 5,26 g Triäthylamin in 50 ml Dimethylsulfoxyd wird während 1 Stunde unter Argon auf 80° erhitzt, und danach wird das abgekühlte Gemisch mit Wasser verdünnt. Der Festkörper wird abfiltriert, in Methylenchlorid gelöst und mit gesättigter Natriumbicarbonatlösung gewaschen, über Natriumsulfat getrocknet, mit Kohle behandelt und eingedampft. Der Rückstand wird aus Aethanol krisallisiert, wobei man 5,8 g 6,7-Dimethoxy-1-/(3,4-dimethoxyphenyl) methyl/-4-/[4-(2-methylphenyl)-1-piperazinyl] methyl/isochinolin als gelben Festkörper erhält, Schmelzpunkt 154-156°. Umkristallisation aus Aethanol liefert eine Analysenprobe vom Schmelzpunkt 154-156°.

Analyse für $C_{32}H_{37}N_3O_4$
Berechnet: C 72,84 H 7,07 N 7,96
Gefunden: C 72,94 H 6,97 N 7,93.

5,2 g der freien Base von 6,7-Dimethoxy-1-/(3,4-dimethoxyphenyl) methyl/-4-/[4-(2-methylphenyl)-1-piperazinyl] methyl/isochinolin in 50 ml warmem Aethanol werden mit 2,2 Moläquivalenten äthanolischer Salzsäurelösung versetzt, und der erhaltene Festkörper abfiltriert. Umkristallisation aus 95 %igem Aethanol liefert 5,3 g analysenreines hydratisiertes Dihydrochloridsalz vom Schmelzpunkt 227-229°.

Analyse für $C_{32}H_{37}N_3O_4 \cdot 2HCl \cdot 1,25\ H_2O$.
Berechnet: C 61,71 H 6,68 N 6,75 Cl 11,38 $H_2O$ 3,61
Gefunden: C 61,43 H 6,36 N 6,64 Cl 11,32 $H_2O$ 3,87.

Beispiel 15

Herstellung von 6,7-Dimethoxy-1-/(3,4-dimethoxyphenyl)-methyl/-4-/[4-(2-methoxyphenyl)-1-piperazinyl] methyl/isochinolin und dessen Dihydrobromidsalz

Ein Gemisch von 10,0 g 4-Chlormethyl-6,7-dimethoxy-1-[(3,4-dimethoxyphenyl) methyl] isochinolin, 5,5 g 1-(2-Methoxyphenyl) piperazin und 7,6 ml Triäthylamin in 100 ml Dimethylsulfoxyd wird während 1 Stunde unter Argon auf 80° erhitzt. Die abgekühlte Lösung wird dann mit Wasser verdünnt und mit Methylenchlorid extrahiert. Die organische Phase wird mit Natriumbicarbonatlösung gewaschen, über

Natriumsulfat getrocknet, mit Kohle behandelt und eingedampft. Der Rückstand wird aus Aethanol kristallisiert, wobei man 11,1 g 6,7-Dimethoxy-1-/(3,4-dimethoxyphenyl) methyl/-4-/[4(2-methoxyphenyl)-1-piperazinyl] methyl/isochinolin als gelben Festkörper erhält, Schmelzpunkt 117-119°.

11,0 g der freien Base von 6,7-Dimethoxy-1-/(3,4-dimethoxyphenyl) methyl/-4-/[4-(2-methoxyphenyl)-1-piperazinyl] methyl/isochinolin in 150 ml warmem Aethanol wird mit 2 Moläquivalenten äthanolischer Bromwasserstoffsäurelösung versetzt, und der erhaltene Festkörper abfiltriert. Umkristallisation aus 95 %igem Aethanol und Trocknen des erhaltenen Festkörpers bei 90°/13 Pa über Phosphorpentoxyd liefert 14,0 g analysenreines Dihydrobromidsalz, Schmelzpunkt 222-223°.

Analyse für $C_{32}H_{37}N_3O_5 \cdot 2HBr$

| | | | | |
|---|---|---|---|---|
| Berechnet : | C 54,48 | H 5,57 | N 5,96 | Br 22,65 |
| Gefunden : | C 54,34 | H 5,33 | N 5,85 | Br 22,73. |

Beispiel 16

Herstellung von 6,7-Dimethoxy-1-/(3,4-dimethoxyphenyl)-methyl/-4-/[4-(4-methoxyphenyl)-1-piperazinyl] methyl/isochinolin und dessen Dihydrochloridsalz

Ein Gemisch von 1,16 g 4-Chlormethyl-6,7-dimethoxy-1-[(3,4-dimethoxyphenyl) methyl] isochinolin, 0,8 g 1-(4-Methoxyphenyl) piperazin-hydrochlorid und 1,3 ml Triäthylamin in 10 ml Dimethylsulfoxyd wird während 1 Stunde unter Argon auf 80° erhitzt. Die abgekühlte Lösung wird danach mit Wasser verdünnt und mit Methylenchlorid extrahiert. Die organische Phase wird mit Natriumbicarbonatlösung gewaschen, über Natriumsulfat getrocknet, mit Kohle behandelt und eingedampft. Der Rückstand wird aus Aethanol kristallisiert, wobei man 1,3 g 6,7-Dimethoxy-1-/(3,4-dimethoxyphenyl) methyl/-4-/[4-(4-methoxyphenyl)-1-piperazinyl] methyl/isochinolin als gelben Festkörper erhält, Schmelzpunkt 176-177°. Umkristallisation aus Aethanol liefert eine Analysenprobe vom Schmelzpunkt 176-177°.

Analyse für $C_{32}H_{37}N_3O_5$

| | | | |
|---|---|---|---|
| Berechnet : | C 70,69 | H 6,86 | N 7,73 |
| Gefunden : | C 70,99 | H 6,81 | N 7,80. |

0,9 g der freien Base von 6,7-Dimethoxy-1-/(3,4-dimethoxyphenyl) methyl/-4-/[4-(4-methoxyphenyl)-1-piperazinyl]-methyl/isochinolin wird in warmem Aethanol aufgeschlämmt und mit 2,2 Moläquivalenten äthanolischer Chlorwasserstoffsäurelösung versetzt, und der erhaltene Festkörper abfiltriert. Umkristallisation aus Methanol/Aethanol liefert analysenreines Dihydrochloridsalz vom Schmelzpunkt 240-241° (Zers.).

Analyse für $C_{32}H_{37}N_3O_5 \cdot 2HCl$

| | | | | |
|---|---|---|---|---|
| Berechnet : | C 62,34 | H 6,38 | N 6,82 | Cl 11,50 |
| Gefunden : | C 62,15 | H 6,26 | N 6,94 | Cl 11,63. |

Beispiel 17

Herstellung von 6,7-Dimethoxy-1-(3,4-dimethoxyphenyl) methyl-4-[(4-phenyl-1-piperidinyl) methyl] isochinolin und dessen Dihydrochlorid-1,75-molares-hydratsalz

Ein Gemisch von 5,0 g 4-Chlormethyl-6,7-dimethoxy-1-[(3,4-dimethoxyphenyl) methyl] isochinolin, 2,1 g 4-Phenyl-piperidin und 2,63 g Triäthylamin in 50 ml Dimethylsulfoxyd wird während 1 Stunde unter Argon auf 80° erhitzt, und dann wird das abgekühlte Reaktionsgemisch mit Wasser verdünnt. Der Festkörper wird abfiltriert, in Methylenchlorid gelöst, mit gesättigter Natriumbicarbonatlösung gewaschen, über Natriumsulfat getrocknet, mit Kohle behandelt und eingedampft. Der Rückstand wird aus Aethanol kristallisiert, wobei man 5,3 g 6,7-Dimethoxy-1-(3,4-dimethoxyphenyl) methyl-4-[(4-phenyl-1-piperidinyl) methyl] isochinolin erhält. Schmelzpunkt 143-145°. Umkristallisation aus Aethanol liefert eine Analysenprobe vom Schmelzpunkt 143-145°.

Analyse für $C_{32}H_{36}N_2O_4$

| | | | |
|---|---|---|---|
| Berechnet : | C 74,97 | H 7,08 | N 5,46 |
| Gefunden : | C 75,19 | H 6,98 | N 5.54 |

5,0 g der freien Base von 6,7-Dimethoxy-1-(3,4-dimethoxyphenyl) methyl-4-[(4-phenyl-1-piperidinyl) methyl] isochinolin in 50 ml warmem Aethanol wird mit 2 Moläquivalenten äthanolischer Salzsäurelösung versetzt, und der erhaltene Festkörper abfiltriert. Umkristallisation aus 95 %igem Aethanol liefert 5,5 g analysenreines hydratisiertes Dihydrochlorsalz vom Schmelzpunkt 223-224°.

Analyse für $C_{32}H_{36}N_2O_4 \cdot 2HCl \cdot 1{,}75\ H_2O$

| | | | | | |
|---|---|---|---|---|---|
| Berechnet : | C 62,28 | H 6,78 | N 4,54 | Cl 11,49 | $H_2O$ 5,10 |
| Gefunden : | C 62,28 | H 6,84 | N 4,40 | Cl 11,64 | $H_2O$ 5,28. |

Beispiel 18

Herstellung von 6,7-Dimethoxy-1-/(3,4-dimethoxyphenyl)-methyl/-4-/[4-(2-methoxyphenyl)-1-piperidinyl]

methyl/isochinolin und dessen Dihydrochlorid-0,3-molares-hydratsalz

Ein Gemisch von 5,0 g 4-Chlormethyl-6,7-dimethoxy-1-[(3,4-dimethoxyphenyl) methyl] isochinolin, 2,49 g 4-(2-Methoxyphenyl) piperidin und 2,63 g Triäthylamin in 50 ml Dimethylsulfoxyd wird während 1 Stunde unter Argon auf 80° erhitzt, und danach wird die abgekühlte Lösung mit Wasser verdünnt und mit Methylenchlorid extrahiert. Die organische Phase wird mit Natriumbicarbonatlösung gewaschen, über Natriumsulfat getrocknet, mit Kohle behandelt und eingedampft, wobei man 7,0 g 6,7-Dimethoxy-1/(3,4-dimethoxyphenyl) methyl/-4-/[4-(2-methoxyphenyl)-1-piperidinyl] methyl/-isochinolin als gelben Schaum erhält.

7,0 g der freien Base von 6,7-Dimethoxy-1-/(3,4-dimethoxyphenyl) methyl/-4-/[4-(2-methoxyphenyl)-1-piperidinyl]-methyl/isochinolin in 50 ml warmem Aethanol wird mit 2,2 Moläquivalenten äthanolischer Salzsäurelösung versetzt, und der erhaltene Festkörper abfiltriert. Umkristallisation aus 95 %igem Aethanol liefert 5,5 g analysenreines hydratisiertes Dihydrochloridsalz vom Schmelzpunkt 229-231° (Zers.).

Analyse für $C_{33}H_{38}N_2O_5 \cdot 2HCl \cdot 0,3\ H_2O$

| | | | | | |
|---|---|---|---|---|---|
| Berechnet : | C 63,83 | H 6,59 | N 4,51 | Cl 11,52 | $H_2O$ 0,86 |
| Gefunden : | C 63,86 | H 6,24 | N 4,55 | Cl 11,29 | $H_2O$ 0,86. |

## Beispiel 19

Herstellung von 1-/[6,7-Dimethoxy-1-[(3,4-dimethoxyphenyl)-methyl]-4-isochinolinyl/methyl/-2-pyrrolidinon und dessen Hydrochloridsalz

Ein Gemisch von 1,4 g 2-Pyrrolidinon, 0,66 g einer 60 %igen Natriumhydriddispersion in Oel und 175 ml getrocknetem und destilliertem Tetrahydrofuran wird während 1 Stunde unter Rühren in einer Argonatmosphäre zum Rückfluss erhitzt. 5,8 g 4-Chlormethyl-6,7-dimethoxy-1-[(3,4-dimethoxyphenyl) methyl] isochinolin werden dann zugegeben und das Erhitzen zum Rückfluss während 18 Stunden fortgesetzt. Eine frische Suspension von Natriumpyrrolidinon (hergestellt aus 0,7 g 2-Pyrrolidinon und 0,33 g 60 %igem Natriumhydrid in Tetrahydrofuran wie oben) wird zugegeben und das Erhitzen zum Rückfluss für weitere 24 Stunden fortgesetzt. Danach wird das Gemisch zur Trockene eingedampft und der Rückstand zwischen Methylenchlorid und Wasser verteilt. Die organische Phase wird über Natriumsulfat getrocknet und eingedampft, und der Rückstand mit absolutem Aethanol extrahiert, mit Kohle versetzt und filtriert. Eindampfen der äthanolischen Lösung liefert 5,3 g rohes 1-//6,7-Dimethoxy-1-[(3,4-dimethoxyphenyl) methyl]-4-isochinolinyl/methyl/-2-pyrrolidinon als gelben Schaum.

Rohes 1-//6,7-Dimethoxy-1-[(3,4-dimethoxyphenyl) methyl]-4-isochinolinyl/methyl/-2-pyrrolidinon (5,3 g) in 50 ml absolutem Aethanol wird mit 1,2 Moläquivalenten äthanolischer Salzsäurelösung versetzt, und das Reaktionsgemisch durch Celit filtriert. Verdünnen mit Aether liefert 4 g eines farblosen Festkörpers, Schmelzpunkt 214-215° (Zers.).

Analyse für $C_{25}H_{28}N_2O_5 \cdot HCl$

| | | | | |
|---|---|---|---|---|
| Berechnet : | C 63,49 | H 6,18 | N 5,92 | Cl 7,50 |
| Gefunden : | C 63,16 | H 6,20 | N 5,76 | Cl 7,58. |

## Beispiel 20

Herstellung von 2-[Bis(phenylmethyl) amino]-N-(2-methoxyphenyl) acetamid

Zu einer gerührten Lösung von 28,1 g 2-Chlor-N-(2-methoxyphenyl) acetamid (C.A. 50, 8730i) in 200 ml Toluol werden 58,3 g Dibenzylamin zugegeben und das Reaktionsgemisch während 48 Stunden zum Rückfluss erhitzt. Nach Abtrennen des Dibenzylamin-hydrochlorids wird das Lösungsmittel aus dem Reaktionsgemisch entfernt. Kristallisation des Rückstandes aus Isopropyläther liefert 41,9 g farblose Kristalle von 2-[Bis(phenylmethyl) amino]-N-(2-methoxyphenyl) acetamid, Schmelzpunkt 92-94°.

## Beispiel 21

Herstellung von N,N-Dibenzyl-N'-(2-methoxyphenyl) äthylendiamin

Zu einer gerührten Aufschlämmung von 11 g Lithiumaluminiumhydrid in 250 ml wasserfreiem Tetrahydrofuran wird unter Stickstoff tropfenweise eine Lösung von 43,6 g 2-[Bis(phenylmethyl) amino]-N-(2-methoxyphenyl) acetamid in 500 ml wasserfreiem Tetrahydrofuran gegeben, und das Reaktionsgemisch bei Raumtemperatur über Nacht gerührt. Nach Abkühlen auf 0° wird das Reaktionsgemisch vorsichtig mit 50 ml Wasser versetzt. Der körnige Niederschlag wird abfiltriert und einige Male mit Methylenchlorid gewaschen. Das Filtrat wird zur Trockene eingedampft, der Rückstand in Aether gelöst, und die organische Phase mit 3N Salzsäure extrahiert. Die wässrige Phase wird mit 3N Natriumhydroxydlösung alkalisch gestellt, und die Base mit Methylenchlorid extrahiert. Die organische Phase wird mit Wasser gewaschen, über Magnesiumsulfat getrocknet und unter vermindertem Druck eingedampft. Der

rohe Sirup (36,9 g) wird an einer Silicagelsäule in Methylenchlorid chromatographiert. Eluieren mit Methylenchlorid liefert N,N-Dibenzyl-N'-(2-methoxyphenyl) äthylendiamin (22,8 g) in Form eines Oels.

Beispiel 22

Herstellung von 4-Benzyl-1-(2-methoxyphenyl)-2-piperazinon

Zu einer gerührten Lösung von 21,6 g N,N-Dibenzyl-N'-(2-methoxyphenyl) äthylendiamin in 400 ml Methylenchlorid werden 12,9 g Natriumcarbonat gegeben. Zu dieser Mischung wird tropfenweise eine Lösung von 8,42 g Chloracetylchlorid in 100 ml Methylenchlorid innerhalb von 30 Minuten gegeben und danach für 1 Stunde gerührt. 500 ml Wasser werden zugegeben, und die organische Phase abgetrennt, mit Wasser gewaschen, über Magnesiumsulfat getrocknet und unter vermindertem Druck eingedampft. Der erhaltene Rückstand (27,9 g) wird in einem Kolben in ein Oelbad von 180° gebracht, und das gebildete Benzylchlorid abdestilliert. Das Rohprodukt wird an 250 g Silicagel unter Verwendung von Aether als Eluierungsmittel chromatographiert. Die passenden Fraktionen werden zur Trockene eingedampft, und der Rückstand aus Isopropyläther umkristallisiert, wobei man 11,0 g 4-Benzyl-1-(2-methoxyphenyl)-2-piperzinon in Form von gelblichen Kristalle erhält, Schmelzpunkt 109-111°, dünnschichtchromatographisch rein (Aether/Silicagel).

Beispiel 23

Herstellung von 4-(2-Methoxyphenyl)-3-piperazinon-hydrochlorid

Zu einer Lösung von 9.4 g 4-Benzyl-1-(2-methoxy-phenyl)-2-piperazinon in 100 ml Aethanol wird 1 g 5 %iges Palladium auf Kohle gegeben, und das Gemisch bei Raumtemperatur und $1{,}0555 \cdot 10^5$ Pa hydriert. Nach 15,5 Stunden wird der Katalysator abfiltriert, und das Filtrat an einem Rotationsverdampfer bei 40° zu einer gelblichen Flüssigkeit (6,8 g) eingedampft, welche in mit trockenem Chlorwasserstoff gesättigtem, Aethanol gelöst wird. Nach dem Eindampfen wird das Rohrprodukt aus Methanol/Aether kristallisiert, wobei man gelbliche Kristalle von 4-(2-Methoxy-phenyl)-3-piperazinon-hydrochlorid erhält, Schmelzpunkt 209-211°.

Beispiel 24

Herstellung von 4-[(1-(3,4-Dimethoxybenzyl)-6,7-dimethoxy-4-isochinolinyl)methyl]-1-(2-methoxyphenyl)-2-piperazinon und dessen Hydrochlorid mit 1,25 Mol Chlorwasserstoff

Ein Gemisch von 6,4 g der freien Base von 4-(2-Methoxyphenyl)-3-piperazinon, 100 ml Dimethylformamid, 8,4 ml Triäthylamin und 11,1 g 4-Chlormethyl-6,7-dimethoxy-1-[(3,4-dimethoxyphenyl)methyl] isochinolin wird unter Rühren während 6 Stunden auf 50° erhitzt. Nach Abdestillieren des Lösungsmittels wird der Rückstand mit 100 ml Wasser gemischt und dreimal mit je 150 ml Methylenchlorid extrahiert. Die organische Phase wird gut mit Wasser gewaschen, über Magnesiumsulfat getrocknet und unter vermindertem Druck eingedampft, wobei man 18,9 g eines Rückstands erhält, der nach Anreiben mit Aethanol kristallisiert. Nach Umkristallisieren aus heissem Aethanol erhält man 12,5 g farblose Kristalle von 4-[(1-(3,4-Dimethoxybenzyl)-6,7-dimethoxy-4-isochinolinyl)methyl]-1-(2-methoxyphenyl)-2-piperazinon, Schmelzpunkt 121-123°, dünnschichtchromatographisch rein (Aethanol/Silicagel).

$C_{32}H_{35}N_3O_6 \cdot 0{,}5C_2H_5OH$ (580,68)
Berechnet : C 68,3 H 6,6 N 7,2
Gefunden : C 68,2 H 6,7 N 7,4.

Die obige Base liefert nach Behandlung mit äthanolischer Salzsäure 4-[(1-(3,4-Dimethoxybenzyl)-6,7-dimethoxy-4-isochinolinyl)methyl]-1-(2-methoxyphenyl)-2-piperazinon-hydrochlorid, welches nach Umkristallisation aus Aethanol bei 237-239° unter Zersetzung schmilzt.

$C_{32}H_{35}N_3O_6 \cdot 1{,}25HCl$ (603,22)
Berechnet : C 63,7 H 6,1 N 7,0 Cl 7,3
Gefunden : C 63,7 H 6,0 N 6,9 Cl 7,2.

Beispiel 25

Herstellung von tert-Butyl-4-(2-methoxyphenyl)-1-piperazincarboxylat

Zu einer Lösung von 36,4 g 1-(2-Methoxyphenyl)piperazin in 500 ml Methylenchlorid (wasserfrei) wird innerhalb von 45 Minuten unter Rühren und unter Stickstoff eine Lösung von 48 g di-tert-Butyldicarbonat in 300 ml Methylenchlorid (wasserfrei) gegeben. Nach Rühren über Nacht bei Raumtemperatur wird das Lösungsmittel unter vermindertem Druck entfernt. Der gelbe Rückstand wird in 300 ml n-Hexan gelöst und auf — 20° abgekühlt. Nach 3 Stunden werden die farblosen Kristalle abfiltriert, wobei man 35,6 g tert-Butyl-4-(2-methoxyphenyl)-1-piperazincarboxylat erhält, Schmelzpunkt 70-71°.

Beispiel 26

Herstellung von tert-Butyl-4-(2-methoxyphenyl)-1-piperazincarboxylat-4-oxyd

Zu einer eisgekühlten Lösung von 35,6 g tert-Butyl-4-(2-methoxyphenyl)-1-piperazincarboxylat in 450 ml Methylenchlorid wird unter Rühren und unter Stickstoff innerhalb von 3,5 Stunden eine Lösung von 28,8 g m-Chlorperbenzoesäure in 450 ml Methylenchlorid in der Weise zugegeben, dass die Temperatur 3-5° nicht übersteigt. Nach weiteren 3 Stunden wird die Hälfte des Lösungsmittels abdestilliert, und die Lösung über 350 g neutrales Aluminiumoxyd filtriert. Nach Eindampfen des Lösungsmittels (2 000 ml Methylenchlorid und 1 500 ml Methylenchlorid enthaltend 5 % Methanol) wird der Rückstand durch Anreiben mit Isopropyläther kristallisiert, wobei man 33,1 g farblose hygroskopische Kristalle von tert-Butyl-4-(2-methoxyphenyl)-1-piperazincarboxylat-4-oxyd erhält, Schmelzpunkt 136-137°.

Beispiel 27

Herstellung von 1-(2-Methoxyphenyl)piperazin-1-oxyd

Zu einer gerührten eiskalten Lösung von 260 ml Trifluoressigsäure werden unter Stickstoff 26 g tert-Butyl-4-(2-methoxyphenyl)-1-piperazincarboxylat-4-oxyd gegeben und bei Raumtemperatur für weitere 6 Stunden gerührt. Das Lösungsmittel wird unter vermindertem Druck entfernt. Die Aufschlämmung wird in 200 ml Aether aufgenommen und zweimal mit je 150 ml Wasser extrahiert. Die wässrige Lösung wird auf eine mit 500 g Dowex 2 (× 4, 20-50 mesh, $OH^-$-Form) gegeben, und die N-Oxydbase mit Wasser eluiert. Nach Eindampfen des Lösungsmittels unter vermindertem Druck wird der weisse Schaum (18,6 g) in einer Minimalmenge Methylenchlorid gelöst, und die Base durch Zugabe von Diisopropyläther ausgefällt, wobei man 13,1 g farblose hygroskopische Kristalle von 1-(1-Methoxyphenyl)piperazin-1-oxid erhält, Schmelzpunkt 157-160°. Das Dihydrochlorid wird aus Aethanol/Aether kristallisiert, Schmelzpunkt 192-194°.

Beispiel 28

Herstellung von 1-(3,4-Dimethoxybenzyl)-6,7-dimethoxy-4-[4'-(2-methoxyphenyl)-1'-piperazinyl]methyl isochinolin-4'-oxid

Ein Gemisch von 1,72 g 4-Chlormethyl-6,7-dimethoxy-1-[(3,4-dimethoxyphenyl)methyl]isochinolin, 20 ml Dimethylformamid, 1,3 ml Triäthylamin und 1,0 g 1-(2-Methoxyphenyl)piperazin-1-oxyd wird unter einer Argonatmosphäre während 4 Stunden bei 50° gerührt. Die abgekühlte Lösung wird in Eiswasser gegossen, dreimal mit Methylenchlorid extrahiert, und die organischen Phasen ihrerseits mit Wasser, gesättigter Natriumbicarbonatlösung und wiederum Wasser gewaschen. Die vereinigten organischen Phasen werden über Magnesiumsulfat getrocknet, und das Lösungsmittel unter vermindertem Druck abgedampft. Die erhaltene kristalline Base wird aus Methylenchlorid/Isopropyläther umkristallisiert, wobei man farblose Kristalle von 1-(3,4-Dimethoxybenzyl)-6,7-dimethoxy-4-/[4'-(2-methoxyphenyl)-1'-piperazinyl]methyl/isochinolin-4'-oxyd erhält, Schmelzpunkt 205-206° (Zers.), dünnschichtchromatographisch rein (Methanol/Silicagel : UV).

$C_{32}H_{37}N_3O_6$ (559,66)
Berechnet : C 68,7 H 6,7 N 7,5
Gefunden : C 68,4 H 6,8 N 7,4.

Das Trihydrochlorid schmilzt nach Kristallisation aus Aethanol unter Zersetzung bei 208-210°.
$C_{32}H_{37}N_3O_6 \cdot 3HCl$ (669,05)
Berechnet : C 57,4 H 6,0 N 6,3
Gefunden : C 57,3 H 6,2 N 6,3.

Beispiel 29

Herstellung von 6,7-Dimethoxy-4-/[4'-(2-methoxyphenyl)-1'-piperazinyl]methyl/isochinolin-1',4'-dioxyd

Zu einer eisgekühlten Lösung von 10,86 g 6,7-Dimethoxy-1-[(3,4-dimethoxyphenyl)methyl]-4-[[4-(2-methoxyphenyl)-1-piperazinyl]methyl]isochinolin in 100 ml Methylenchlorid wird unter Rühren und unter Stickstoff eine Lösung von 10,35 g m-Chlorperbenzoesäure in 200 ml Methylenchlorid innerhalb von 1 3/4 Stunden in der Weise zugegeben, dass die Temperatur 3° nicht übersteigt. Nach 1,5 Stunden werden 300 ml Methylenchlorid zugegeben, und die organische Phase ihrerseits mit Wasser, gesättigter Natriumbicarbonatlösung und nochmals Wasser gewaschen, über Magnesiumsulfat getrocknet, und das Lösungsmittel unter vermindertem Druck abgedampft. Das rohe gelbe kristalline Produkt wird aus Methylenchlorid/Isopropyläther kristallisiert, wobei man 5,2 g farblose Kristalle von 6,7-Dimethoxy-4-/[4'-(2-methoxyphenyl)-1'-piperazinyl]methyl/isochinolin-1',4'-dioxyd erhält, Schmelzpunkt 186-188° (Zers.).

$C_{32}H_{37}N_3O_7$ (575,66)
Berechnet : C 67,1 H 6,6 N 7,2
Gefunden : C 66,8 H 6,5 N 7,3.

Beispiel 30

Herstellung von 1-(3,4-Dimethoxybenzyl)-6,7-dimethoxy-4-/[4'-(2-methoxyphenyl)-1'-piperazinyl]methyl/isochinolin-1'-oxid

Zu einer eisgekühlten Lösung von 33,0 g 6,7-Dimethoxy-1-[(3,4-dimethoxyphenyl)methyl]-4-[[4-(2-methoxyphenyl)-1-piperzinyl]methyl]isochinolin in 500 ml Methylenchlorid wird unter Stickstoff und unter Rühren eine Lösung von 10,4 g m-Chlorperbenzoesäure in 200 ml Methylenchlorid innerhalb von 3 Stunden bei 0-3° zugegeben. Die Lösung wird für weitere 20 Stunden bei 0° gerührt und danach auf eine mit 700 g Silicagel 60 Merck (230-400 mesh ASTM) beladene Säule gegeben. Eluieren mit Aethylacetat liefert 8,7 g unreagiertes 6,7-Dimethoxy-1-[3,4-dimethoxyphenyl)methyl]-4-[[4-(2-methoxyphenyl)-1-piperazinyl]methyl]isochinolin. Weiteres Eluieren mit Aethylacetat/30 % Aethanol liefert 7,1 g eines Produktes, welches aus Methylenchlorid/Isopropyläther kristallisiert wird und 5,7 g farblose hygroskopische Kristalle von 1-(3,4-Dimethoxybenzyl)-6,7-dimethoxy-4/[4'-(2-methoxyphenyl)-1'-piperazinyl]methyl]/isochinolin-1'-oxyd liefert, Schmelzpunkt 161-162°.

$C_{32}H_{37}N_3O_6$ (559,66)
Berechnet : C 68,7 H 6,7 N 7,5
Gefunden : C 68,8 H 7,0 N 7,3.

Weiteres Eluieren mit Aethylacetat/70 % Aethanol liefert Fraktionen von reinem 1-(3,4-Dimethoxybenzyl)-6,7-dimethoxy-4-/[4'-(2-methoxyphenyl-1'-piperazinyl]methyl/isochinolin-4'-oxyd, Schmelzpunkt 204-206° aus Methylenchlorid/Isopropyläther (0,9 g).

Beispiel 31

Herstellung von 4-(Chlormethyl)-1-(3,4-dimethoxybenzyl)-6,7-dimethoxyisochinolin-2-oxyd

Zu einer eisgekühlten Lösung von 5,2 g 4-Chlormethyl-6,7-dimethoxy-1-[(3,4-dimethoxyphenyl)methyl]isochinolin in 150 ml Methylenchlorid wird unter Rühren und unter Stickstoff eine Lösung von 3,04 g m-Chlorperbenzoesäure in 100 ml Methylenchlorid innerhalb von 2 Stunden in der Weise zugegeben, dass die Temperatur 3-5° nicht übersteigt. Die Lösung wird über Nacht bei Raumtemperatur gerührt, und danach wird die rote Lösung auf eine mit 100 g Silicagel 60 Merck (230-400 mesh ASTM) beladene Säule gegeben. Durch Eluieren mit Aether und Aethylacetat wird unreagierte Base von 4-Chlormethyl-6,7-dimethoxy-1-[(3,4-dimethoxyphenyl)-methyl]isochinolin abgetrennt. Eluieren mit Aethylacetat/Aethanol (4 : 1) liefert 3,8 g eines bräunlichen Schaums. Das 4-(Chlormethyl)-1-(3,4-dimethoxybenzyl)-6,7-dimethoxyisochinolin-2-oxyd kristallisiert aus Methylenchlorid/Aethylacetat : 3,5 g gelbe Kristalle, Schmelzpunkt 167-168°.

Beispiel 32

Herstellung von 1-(3,4-Dimethoxybenzyl)-6,7-dimethoxy-4-/[4'-(2-methoxyphenyl)-1'-piperazinyl]methyl/isochinolin-2-oxyd

Ein Gemisch von 2,5 g 4-Chlormethyl-1-(3,4-dimethoxybenzyl)-6,7-dimethoxyisochinolin-2-oxyd, 35 ml Dimethylformamid, 1,8 ml Triäthylamin und 1,29 g 1-(2-Methoxyphenyl)piperazin wird während 7 Stunden unter Stickstoff gerührt und auf 50° erhitzt. Nach dem Abdampfen des Lösungsmittels wird der Rückstand zwischen Methylenchlorid und Wasser verteilt. Die Methylenchloridlösung wird mit Wasser gewaschen und getrocknet. Nach dem Entfernen des Lösungsmittels unter vermindertem Druck wird der Rückstand (3,5 g) aus Methylenchlorid/Aethylacetat kristallisiert, wobei man 3,05 g gelblicher, hygroskopischer Kristalle von 1-(3,4-Dimethoxybenzyl)-6,7-dimethoxy-4-/[4'-(2-methoxyphenyl)-1'-piperazinyl]methyl/isochinolin-2-oxyd erhält, Schmelzpunkt 128-129° (Zers.).

$C_{32}H_{37}N_3O_6$ (559,66)
Berechnet : C 68,7 H 6,7 N 7,5
Gefunden : C 68,7 H 6,9 N 7,4.

Das Dihydrochloridsalz schmilzt nach Kristallisation aus Aethanol/Aether unter Zersetzung bei 202-204°.

$C_{32}H_{37}N_3O_6 \cdot 2\ HCl$ (632,59)
Berechnet : C 60,8 H 6,1 N 6,7 Cl 11,4
Gefunden : C 61,1 H 6,1 N 6,7 Cl 11,4.

## Beispiel 33

Herstellung von 1-(3,4-Dimethoxybenzyl)-6,7-dimethoxy-4-[4'-(2-methoxyphenyl)-1'-piperazinyl]methyl/isochinolin-2,4'-dioxyd

Ein Gemisch von 582 mg 4-Chlormethyl-1-(3,4-dimethoxybenzyl)-6,7-dimethoxyisochinolin-2-oxyd, 10 ml Dimethylformamid, 0,4 ml Triäthylamin und 0,3 g 1-(2-Methoxyphenyl)piperazin-1-oxyd wird wie in Beispiel 32 für die Herstellung von 1-(3,4-Dimethoxybenzyl)-6,7-dimethoxy-4-/[4'-(2-methoxyphenyl)-1'-piperazinyl]methyl/isochinolin-2-oxyd beschrieben, umgesetzt. Nach dem Eindampfen des Lösungsmittels wird der Rückstand an 10 g Silicagel chromatographiert. Eluieren mit Aethylacetat/Aethanol (2 : 3) liefert 180 mg eines bräunlichen Schaums, welcher aus Aethylacetat/Aether kristallisiert und 1-(3,4-Dimethoxybenzyl)-6,7-dimethoxy-4-/[4'-(2-methoxyphenyl)-1'-piperazinyl]methyl/isochinolin-2,4'-dioxyd vom Schmelzpunkt 161-163° liefert, welches im Dünnschichtchromatogramm (Methanol/Silicagel : UV) von 6,7-Dimethoxy-4-/[4'-(2-methoxyphenyl)-1'-piperazinyl]methyl/isochinolin-1',4'-dioxyd verschieden ist.

$C_{32}H_{37}N_3O_7$ (575,66)
Berechnet : C 66,8 H 6,5 N 7,3
Gefunden : C 66,5 H 6,5 N 7,1.

## Beispiel A

Tablettenformulierung (Direkte Verpressung)

| | Bestandteile | mg/Tablette | | |
|---|---|---|---|---|
| 1. | 6,7-Dimethoxy-1-[(3,4-dimethoxyphenyl)methyl]-4-[[4-(2-methoxyphenyl)-1-piperazinyl]methyl]isochinolin | 15 | 30 | 60 |
| 2. | Milchzucker | 207 | 192 | 162 |
| 3. | Avicel | 45 | 45 | 45 |
| 4. | Stärke zum Direktverpressen | 30 | 30 | 30 |
| 5. | Magnesiumstearat | 3 | 3 | 3 |
| | Tablettengewicht | 300 mg | 300 mg | 300 mg |

Verfahren

Zum Bestandteil 1 wird die gleiche Menge Milchzucker gegeben und das Ganze gut vermischt. Die Bestandteile 3 und 4 sowie der verbleibende Milchzucker werden ebenfalls zugegeben und gut vermischt. Danach gibt man das Magnesiumstearat zu und mischt für 3 Minuten. Schliesslich wird auf einer geeigneten Maschine zu Tabletten verpresst.

## Beispiel B

Tablettenformulierung (Feuchte Granulation)

| | Bestandteile | mg/Tablette | | |
|---|---|---|---|---|
| 1. | 6,7-Dimethoxy-1-[(3,4-dimethoxyphenyl)methyl]-4-[[4-(2-methoxyphenyl)-1-piperazinyl]methyl] isochinolin | 50 | 100 | 200 |
| 2. | Milchzucker | 153 | 187 | 171 |
| 3. | Modifizierte Stärke | 25 | 35 | 45 |
| 4. | Vorgelatinisierte Stärke | 20 | 25 | 30 |
| 5. | destilliertes Wasser q.s. | - | - | - |
| 6. | Magnesiumstearat | 2 | 3 | 4 |
| | Tablettengewicht | 250 mg | 350 mg | 450 mg |

Verfahren

Die Bestandteile 1-4 werden in einem geeigneten Mixer vermischt. Das Ganze wird danach mit genügend destilliertem Wasser zu einem Granulat geeigneter Konsistenz verarbeitet und vermahlen. Danach trocknet man das Ganze in einem geeigneten Ofen, vermahlt nochmals und mischt während 3 Minuten das Magnesiumstearat zu. Danach wird auf einer geeigneten Presse zu Tabletten verpresst.

Beispiel C

Kapselformulierung

| | Bestandteile | mg/Kapsel | | |
|---|---|---|---|---|
| 1. | 6,7-Dimethoxy-1-[(3,4-dimethoxyphenyl)methyl]-4-[[4-(2-methoxy-phenyl)-1-piperazinyl]methyl]-isochinolin | 50 | 100 | 200 |
| 2. | Milchzucker | 155 | 200 | 140 |
| 3. | Stärke | 30 | 35 | 40 |
| 4. | Talk | 15 | 15 | 20 |
| | Kapselfüllgewicht | 250 mg | 350 mg | 400 mg |

Verfahren

Die Bestandteile 1-3 werden in einem geeigneten Mixer vermischt. Der Talk wird zugegeben, worauf man nochmals vermischt. Danach wird auf einer geeigneten Maschine in Kapseln abgefüllt.

Beispiel D

Kapselformulierung

| | Bestandteile | mg/Kapsel | | |
|---|---|---|---|---|
| 1. | 6,7-Dimethoxy-1-[(3,4-dimethoxyphenyl)methyl]-4-[[4-(2-methoxy-phenyl)-1-piperazinyl]methyl]-isochinolin | 15 | 30 | 60 |
| 2. | Milchzucker | 239 | 224 | 194 |
| 3. | Stärke | 30 | 30 | 30 |
| 4. | Talk | 15 | 15 | 15 |
| 5. | Magnesiumstearat | 1 | 1 | 1 |
| | Kapselfüllgewicht | 300 mg | 300 mg | 300 mg |

Verfahren

Die Bestandteile 1-3 werden in einem geeigneten Mixer vermischt. Der Talk und das Magnesiumstearat werden zugegeben, worauf man nochmals für kurze Zeit gut vermischt. Danach wird auf einer geeigneten Maschine in Kapseln abgefüllt.

Beispiel E

Tablettenformulierung (feuchte Granulation)

| | Bestandteile | mg/Tablette | | |
|---|---|---|---|---|
| 1. | 6,7-Dimethoxy-1-[(3,4-dimethoxyphenyl)methyl]-4-[[4-(2-methoxy-phenyl)-1-piperazinyl]methyl]-isochinolin | 15 | 30 | 60 |

(Fortsetzung)

| | Bestandteile | mg/Kapsel | | |
|---|---|---|---|---|
| 2. | Milchzucker | 188 | 173 | 188 |
| 3. | Modifizierte Stärke | 25 | 25 | 30 |
| 4. | Vorgelatinisierte Stärke | 20 | 20 | 20 |
| 5. | destilliertes Wasser q.s. | - | - | - |
| 6. | Magnesiumstearat | 2 | 2 | 2 |
| | Tablettengewicht | 250 mg | 250 mg | 300 mg |

Verfahren

Die Bestandteile 1-4 werden in einem geeigneten Mixer vermischt. Danach wird das Ganze mit genügend destilliertem Wasser zu einem Granulat geeigneter Konsistenz verarbeitet und vermahlen. Nach dem Trocknen in einem geeigneten Ofen wird das Ganze nochmals gemahlen und mit dem Magnesiumstearat vermischt. Danach wird auf einer geeigneten Presse zu Tabletten verpresst.

**Patentansprüche** (für die Vertragsstaaten : BE, CH, DE, FR, GB, IT, LI, LU, NL, SE)

1. Eine Verbindung der allgemeinen Formel

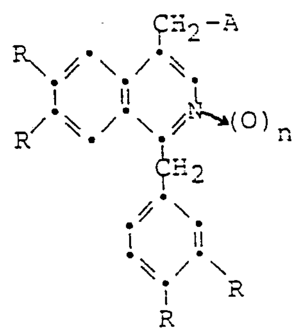

(I)

worin R nieder Alkoxy, n die Zahl 0 oder 1 und A eine der Gruppen

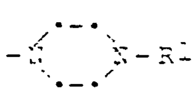

(a),

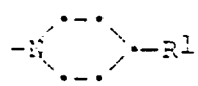

(b),

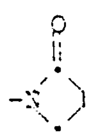

(c),

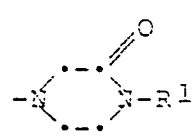

(d),

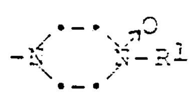

(e),

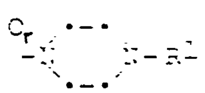

(f) und

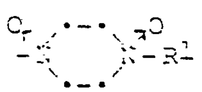

(g)

bedeuten, worin $R^1$ Phenyl, Halophenyl, nieder Alkylphenyl oder nieder Alkoxyphenyl bedeutet, wobei die als « nieder » bezeichneten Gruppen 1-7 Kohlenstoffatome aufweisen und pharmazeutisch verwendbare Säureadditionssalze davon.

2. Eine Verbindung gemäss Anspruch 1, dadurch gekennzeichnet, dass n die Zahl 0 bedeutet.

3. Eine Verbindung gemäss Anspruch 1 oder 2, dadurch gekennzeichnet, dass A die Gruppe

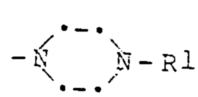

(a)

bedeutet.

4. Eine Verbindung gemäss Anspruch 1 oder 2, dadurch gekennzeichnet, dass A die Gruppe

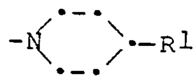

(b)

bedeutet.

5. Eine Verbindung gemäss Anspruch 3 oder 4, dadurch gekennzeichnet, dass $R^1$ nieder Alkoxyphenyl bedeutet.

6. 6,7-Dimethoxy-1-/(3,4-dimethoxyphenyl)methyl/-4-/[4-(2-methoxyphenyl)-1-piperazinyl]methyl/isochinolin.

7. 6,7-Dimethoxy-1-/(3,4-dimethoxyphenyl)methyl/-4-/[4-(2-methoxyphenyl)-1-piperidinyl]methyl/isochinolin.

8. Eine Verbindung gemäss einem der Ansprüche 1-7 oder ein pharmazeutisch verwendbares Säureadditionssalz davon zur Verwendung als pharmazeutischer Wirkstoff.

9. Eine Verbindung gemäss einem der Ansprüche 1-7 oder ein pharmazeutisch verwendbares Säureadditionssalz davon zur Verwendung bei der Behandlung oder Prophylaxe von cerebrovasculären Störungen und Hypertension.

10. Verfahren zur Herstellung von Verbindungen der in Anspruch 1 angegebenen Formel I und von pharmazeutisch verwendbaren Säureadditionssalzen davon, dadurch gekennzeichnet, dass man

a) zur Herstellung von Verbindungen der Formel I, worin A eine der obigen Gruppen (a), (b), (c), (d) und (e) bedeutet, eine Verbindung der allgemeinen Formel

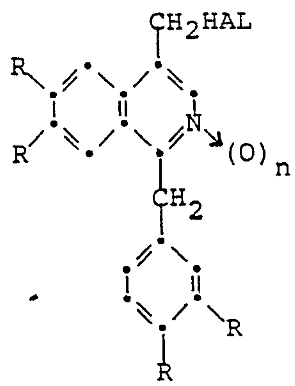

(II)

worin R und n die obige Bedeutung besitzen, mit einem Amin der allgemeinen Formel

$$HA' \quad (III)$$

worin A' eine der obigen Gruppen (a), (b), (c), (d) und (e) bedeutet, umsetzt, oder

b) zur Herstellung von Verbindungen der Formel I, worin A eine der obigen Gruppen (e), (f) und (g) bedeutet, eine erhaltene Verbindung der Formel I, worin A die Gruppe (a) bedeutet, oxidiert und die erhaltenen N-Oxide aus dem Gemisch auftrennt, und

c) erwünschtenfalls, eine erhaltene Verbindung der Formel I in ein pharmazeutisch verwendbares Säureadditionssalz überführt.

11. Arzneimittel enthaltend eine Verbindung gemäss einem der Ansprüche 1-7 oder ein pharmazeutisch verwendbares Säureadditionssalz davon und ein therapeutisch inertes Excipiens.

12. Arzneimittel zur Verwendung bei der Behandlung und Prophylaxe von cerebrovasculären Störungen und Hypertension enthaltend eine Verbindung gemäss einem der Ansprüche 1-7 oder ein pharmazeutisch verwendbares Säureadditionssalz davon und ein therapeutisch inertes Excipiens.

**Patentansprüche** (für den Vertragsstaat AT)

1. Verfahren zur Herstellung von Verbindungen der allgemeinen Formel

(Siehe Formel Seite 26 f.)

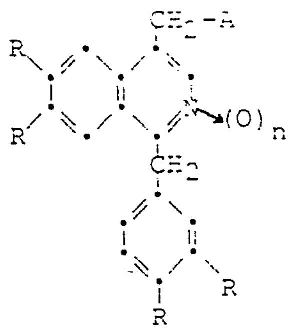

(I)

worin R nieder Alkoxy, ni die Zahl 0 oder 1 und A eine der Gruppen

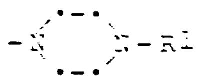

(a),

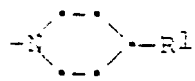

(b),

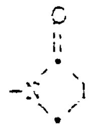

(c),

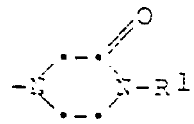

(d),

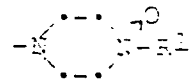

(e),

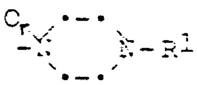

(f) und

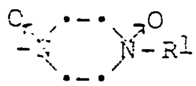

(g)

worin R[1] Phenyl, Halophenyl, nieder Alkylphenyl oder nieder Alkoxyphenyl bedeutet, wobei die als « nieder » bezeichneten Gruppen 1-7 Kohlenstoffatome aufweisen und pharmazeutisch verwendbaren Säureadditionssalzen davon, dadurch gekennzeichnet, das man

a) zur Herstellung von Verbindungen der Formel I, worin A eine der obigen Gruppen (a), (b), (c), (d) und (e) bedeutet, eine Verbindung der allgemeinen Formel

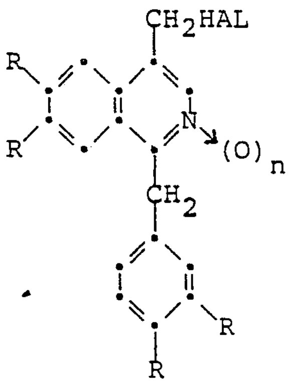

(II)

worin R und n die obige Bedeutung besitzen, mit einem Amin der allgemeinen Formel

HA' (III)

worin A' eine der obigen Gruppen (a), (b), (c), (d) und (e) bedeutet, umsetzt, oder

b) zur Herstellung von Verbindungen der Formel I, worin A eine der obigen Gruppen (e), (f) und (g) bedeutet, eine erhaltene Verbindung der Formel I, worin A die Gruppe (a) bedeutet, oxidiert und die erhaltenen N-Oxide aus dem Gemisch auftrennt, und

c) erwünschtenfalls, eine erhaltene Verbindung der Formel I in ein pharmazeutisch verwendbares Säureadditionssalz überführt.

2. Verfahren nach Anspruch 1, dadurch gekennzeichnet, dass n die Zahl 0 bedeutet.

3. Verfahren nach Anspruch 1 oder 2, dadurch gekennzeichnet, dass A die Gruppe

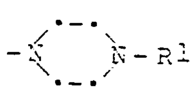

(a)

bedeutet.

4. Verfahren nach Anspruch 1 oder 2, dadurch gekennzeichnet, dass A die Gruppe

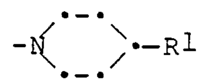

(b)

bedeutet.

5. Verfahren nach Anspruch 3 oder 4, dadurch gekennzeichnet, dass $R^1$ nieder Alkoxyphenyl bedeutet.

6. Verfahren nach Anspruch 1, dadurch gekennzeichnet, dass man 6,7-Dimethoxy-1-/(3,4-dimethoxyphenyl)methyl/-4-/[4-(2-methoxyphenyl)-1-piperazinyl]methyl/isochinolin herstellt.

7. Verfahren nach Anspruch 1, dadurch gekennzeichnet, dass man 6,7-Dimethoxy-1-/(3,4-dimethoxyphenyl)methyl/-4-/[4-(2-methoxyphenyl)-1-piperidinyl]methyl/isochinolin herstellt.

**Claims** (for the Contracting States : BE, CH, DE, FR, GB, IT, LI, LU, NL, SE)

1. A compound of the general formula

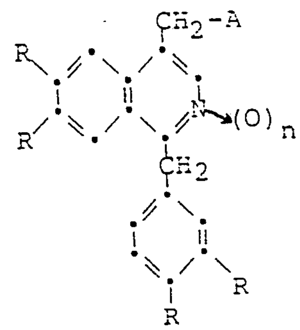

(I)

wherein R signifies lower alkoxy, n signifies the number 0 or 1 and A signifies one of the groups

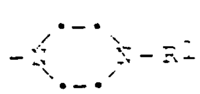

(a),

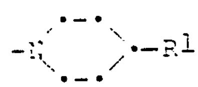

(b),

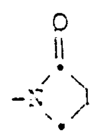

(c),

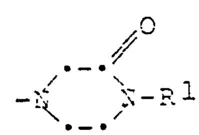

(d),

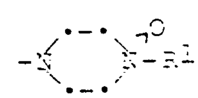

(e),

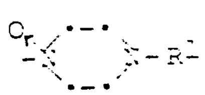

(f) and

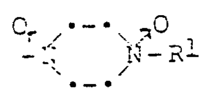

(g)

in which $R^1$ signifies phenyl, halophenyl, lower alkylphenyl or lower alkoxyphenyl, whereby the groups denoted as « lower » have 1-7 carbon atoms, and pharmaceutically usable acid addition salts thereof.

2. A compound in accordance with claim 1, characterized in that n signifies the number 0.

3. A compound in accordance with claim 1 or 2, characterized in that A signifies the group

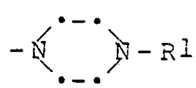

(a)

4. A compound in accordance with claim 1 or 2, characterized in that A signifies the group

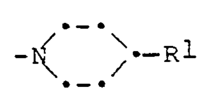

(b)

5. A compound in accordance with claim 3 or 4, characterized in that $R^1$ signifies lower alkoxyphenyl.

6. 6,7-Dimethoxy-1-/(3,4-dimethoxyphenyl)methyl/-4-/[4-(2-methoxyphenyl)-1-piperazinyl]methyl/isoquinoline.

7. 6,7-Dimethoxy-1-/(3,4-dimethoxyphenyl)methyl/-4-/[4-(2-methoxyphenyl)-1-piperidinyl]methyl/isoquinoline.

8. A compound in accordance with any one of claims 1-7 or a pharmaceutically usable acid addition salt thereof for use as a pharmaceutically active substance.

9. A compound in accordance with any one of claims 1-7 or a pharmaceutically usable acid addition salt thereof for use in the treatment or prophylaxis of cerebrovascular disorders and hypertension.

10. A process for the manufacture of compounds of formula I given in claim 1 and of pharmaceutically usable acid addition salts thereof, characterized by

a) for the manufacture of compounds of formula I in which A signifies one of the above groups (a), (b), (c), (d) and (e), reacting a compound of the general formula

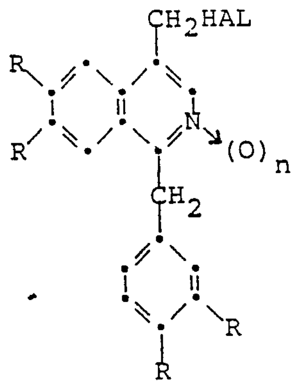

(II)

wherein R and n have the above significance, with an amine of the general formula

HA' (III)

wherein A' signifies one of the above groups (a), (b), (c), (d) and (e), or

b) for the manufacture of compounds of formula I in which A signifies one of the above groups (e), (f) and (g), oxidizing an obtained compound of formula I in which A signifies group (a) and separating the N-oxide obtained from the mixture, and

c) if desired, converting a compound of formula I obtained into a pharmaceutically usable acid addition salt.

11. A medicament containing a compound in accordance with any one of claims 1-7 or a pharmaceutically usable acid addition salt thereof and a therapeutically inert excipient.

12. A medicament for use in the treatment and prophylaxis of cerebrovascular disorders and hypertension containing a compound in accordance with any one of claims 1-7 or a pharmaceutically usable acid addition salt thereof and a therapeutically inert excipient.

**Claims** (for the Contracting State AT)

1. A process for the manufacture of compounds of the general formula

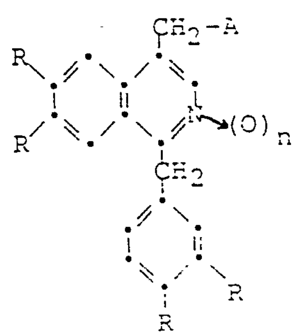

(I)

wherein R signifies lower alkoxy, n signifies the number 0 or 1 and A signifies one of the groups

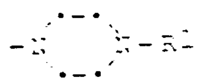 (a), 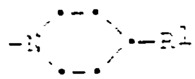 (b), 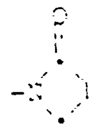 (c), 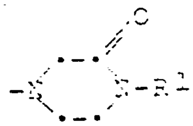 (d), 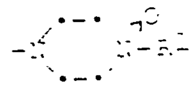 (e),

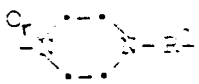 (f) and 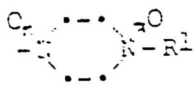 (g)

in which $R^1$ signifies phenyl, halophenyl, lower alkylphenyl or lower alkoxyphenyl, whereby the groups denoted as « lower » have 1-7 carbon atoms, and pharmaceutically usable acid addition salts thereof, characterized by

a) for the manufacture of compounds of formula I in which A signifies one of the groups (a), (b), (c), (d) and (e), reacting a compound of the general formula

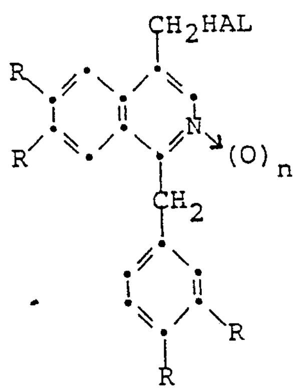

(II)

wherein R and n have the above significance, with an amine of the general formula

HA' (III)

wherein A' signifies one of the above groups (a), (b), (c), (d) and (e), or

b) for the manufacture of compounds of formula I in which A signifies one of the above groups (e), (f) and (g), oxidizing an obtained compound of formula I in which A signifies group (a) and separating the N-oxide obtained from the mixture, and

c) if desired, converting a compound of formula I obtained into a pharmaceutically usable acid addition salt.

2. A process according to claim 1, characterized in that n signifies the number 0.

3. A process according to claim 1 or 2, characterized in that A signifies the group

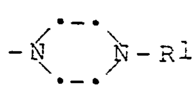

(a)

4. A process according to claim 1 or 2, characterized in that A signifies the group

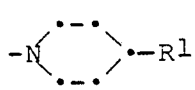

(b)

5. A process according to claim 3 or 4, characterized in that $R^1$ signifies lower alkoxyphenyl.

6. A process according to claim 1, characterized in that 6,7-dimethoxy-1-/(3,4-dimethoxyphenyl) methyl/-4-/[4-(2-methoxyphenyl)-1-piperazinyl] methyl/isoquinoline is manufactured.

7. A process according to claim 1, characterized in that 6,7-dimethoxy-1-/(3,4-dimethoxyphenyl) methyl/4-/[4-(2-methoxyphenyl)-1-piperidinyl] methyl/isoquinoline is manufactured.

**Revendications** (pour les Etats contractants : BE, CH, DE, FR, GB, IT, LI, LU, NL, SE)

1. Un composé de formule générale

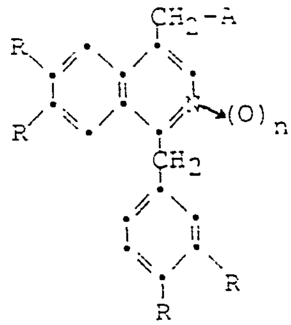

(I)

dans laquelle R représente un groupe alcoxy inférieur, n est égal à 0 ou 1 et A représente l'un des groupes

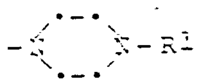

(a),

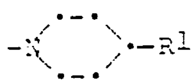

(b),

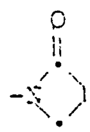

(c),

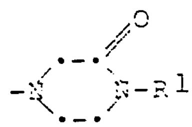

(d),

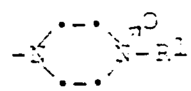

(e),

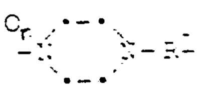

(f) et

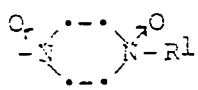

(g)

dans lesquels $R^1$ représente un groupe phényle, halogénophényle, (alkyle inférieur)-phényle ou (alcoxy inférieur)-phényle, les groupes qualifiés d'« inférieurs » contenant 1 à 7 atomes de carbone, et leurs sels acceptables pour l'usage pharmaceutique formés par addition avec des acides.

2. Un composé selon la revendication 1, caractérisé en ce que n est égal à 0.

3. Un composé selon la revendication 1 ou 2, caractérisé en ce que A représente le groupe

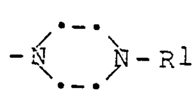

(a)

4. Un composé selon la revendication 1 ou 2, caractérisé en ce que A représente le groupe

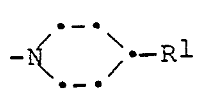

(b)

5. Un composé selon la revendication 3 ou 4, caractérisé en ce que $R^1$ représente un groupe (alcoxy inférieur)-phényle.

6. La 6,7-diméthoxy-1-[(3,4-diméthoxyphényl)-méthyl]-4-{[4-(2-méthoxyphényl)-1-pipérazinyl]-méthyl}-isoquinoléine.

7. La 6,7-diméthoxy-1-[(3,4-diméthoxyphényl)-méthyl]-4-{[4-(2-méthoxyphényl)-1-pipéridinyl]-méthyl}-isoquinoléine.

8. Un composé selon l'une des revendications 1 à 7 ou un sel acceptable pour l'usage pharmaceutique, formé par addition avec un acide, d'un tel composé, pour l'utilisation en tant que substance active pharmaceutique.

9. Un composé selon l'une des revendications 1 à 7 ou un sel acceptable pour l'usage pharmaceutique, formé par addition avec un acide, d'un tel composé, pour l'utilisation dans le traitement ou la prophylaxie des troubles cérébrovasculaires et de l'hypertension.

10. Procédé de préparation des composés de formule I de la revendication 1 et de leurs sels acceptables pour l'usage pharmaceutique formés par addition avec des acides, caractérisé en ce que

a) pour préparer les composés de formule I dans laquelle A représente l'un des groupes a), b), c), d) et e) ci-dessus, on fait réagir un composé de formule générale

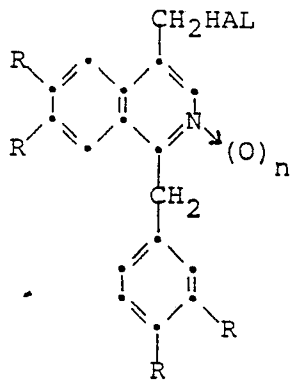

(II)

dans laquelle R et n ont les significations indiquées ci-dessus, avec une amine de formule générale

HA' (III)

dans laquelle A' représente l'un des groupes a), b), c), d) et c) ci-dessus, ou bien

b) pour préparer les composés de formule I dans laquelle A représente l'un des groupes e), f) et g) ci-dessus, on oxyde un composé obtenu, répondant à la formule I dans laquelle A représente le groupe a), et on sépare du mélange des N-oxydes formés, et

c) si on le désire, on convertit un composé obtenu, répondant à la formule I, en un sel acceptable pour l'usage pharmaceutique formé par addition avec un acide.

11. Médicament contenant un composé selon l'une des revendications 1 à 7 ou un sel acceptable pour l'usage pharmaceutique d'un tel composé, formé par addition avec un acide, et un excipient inerte du point de vue thérapeutique.

12. Médicament pour l'utilisation dans le traitement et la prophylaxie des troubles cérébrovasculaires et de l'hypertension, contenant un composé selon l'une des revendications 1 à 7 ou un sel acceptable pour l'usage pharmaceutique d'un tel composé, formé par addition avec un acide, et un excipient inerte du point de vue thérapeutique.

**Revendications** (pour l'Etat contractant AT)

1. Procédé de préparation de composés de formule générale

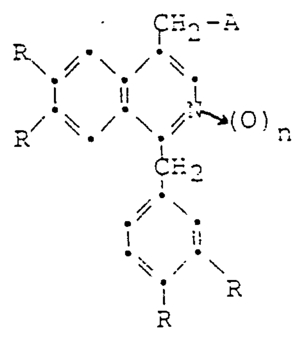

(I)

dans laquelle R représente un groupe alcoxy inférieur, n est égal à 0 ou 1 et A représente l'un des groupes

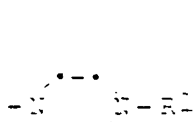
(a),

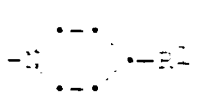
(b),

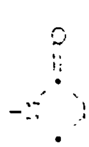
(c),

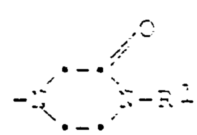
(d),

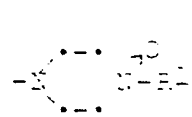
(e),

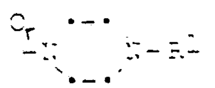

(f) et

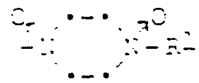

(g)

dans lesquels R[1] représente un groupe phényle, halogénophényle, (alkyle inférieur)-phényle ou (alcoxy inférieur)-phényle, les groupes qualifiés d'« inférieurs » contenant 1 à 7 atomes de carbone, et de leurs sels acceptables pour l'usage pharmaceutique formés par addition avec des acides, caractérisé en ce que

a) pour préparer les composés de formule I dans laquelle A représente l'un des groupes a), b), c), d) et e) ci-dessus, on fait réagir un composé de formule générale

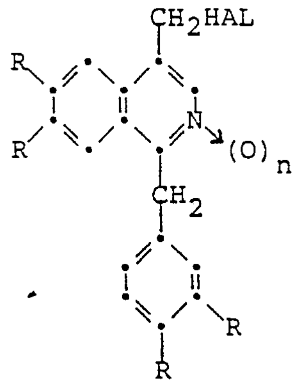

(II)

dans laquelle R et n ont les significations indiquées ci-dessus, avec une amine de formule générale

HA' (III)

dans laquelle A' représente l'un des groupes a), b), c), d) et e) ci-dessus, ou bien

b) pour préparer les composés de formule I dans laquelle A représente l'un des groupes e), f) et g) ci-dessus, on oxyde un composé obtenu, répondant à la formule I dans laquelle A représente le groupe a), et on sépare du mélange les N-oxydes formés, et

c) si on le désire, on convertit un composé obtenu, répondant à la formule I, en un sel acceptable pour l'usage pharmaceutique formé par addition avec un acide.

2. Procédé selon la revendication 1, caractérisé en ce que n est égal à 0.

3. Procédé selon la revendication 1 ou 2, caractérisé en ce que A représente le groupe

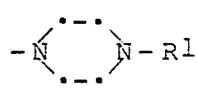

(a)

4. Procédé selon la revendication 1 ou 2, caractérisé en ce que A représente le groupe

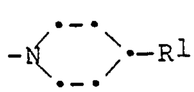

(b)

5. Procédé selon la revendication 3 ou 4, caractérisé en ce que R[1] représente un groupe (alcoxy inférieur)-phényle.

6. Procédé selon la revendication 1, caractérisé en ce que l'on prépare la 6,7-diméthoxy-1-[(3,4-diméthoxyphényl)-méthyl]-4-{[4-(2-méthoxyphényl)-1-pipérazinyl]-méthyl}-isoquinoléine.

7. Procédé selon la revendication 1, caractérisé en ce que l'on prépare la 6,7-diméthoxy-1-[(3,4-diméthoxyphényl)-méthyl]-4-{[4-(2-méthoxyphényl)-1-pipéridinyl]-méthyl}-isoquinoléine.